(19) **Europäisches Patentamt**
**European Patent Office**
**Office européen des brevets**

(11) **EP 4 141 445 A1**

(12) **EUROPEAN PATENT APPLICATION**
published in accordance with Art. 153(4) EPC

(43) Date of publication:
**01.03.2023 Bulletin 2023/09**

(21) Application number: **20931952.4**

(22) Date of filing: **27.05.2020**

(51) International Patent Classification (IPC):
*G01N 33/532* (2006.01)  *G01N 33/574* (2006.01)
*C12N 5/09* (2010.01)

(52) Cooperative Patent Classification (CPC):
**C12N 5/06; G01N 33/53; G01N 33/532;**
**G01N 33/574**

(86) International application number:
**PCT/RU2020/000250**

(87) International publication number:
**WO 2021/215955 (28.10.2021 Gazette 2021/43)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **22.04.2020  RU 2020114411**

(71) Applicant: **JVS Diagnostics Limited Liability**
**Company**
**Moscow, 121205 (RU)**

(72) Inventors:
• **CHERKASOVA, Janneta Rashidovna**
**Moskovskaya obl. 142442 (RU)**
• **TSURKAN, Sergei Alexandrovich**
**Moscow, 121099 (RU)**
• **KONDRATIEV, Vyacheslav Borisovich**
**Moscow, 117513 (RU)**
• **MORO-VIDAL, Ricardo**
**Richmond  BC, V6X 1X7 (CA)**

(74) Representative: **Tombling, Adrian George**
**Withers & Rogers LLP**
**2 London Bridge**
**London SE1 9RA (GB)**

(54) **CANCER ANTIGEN FOR EARLY CANCER DETECTION**

(57)    The invention relates to obtaining, isolating and detecting a new marker of epithelial cancers. A method comprises obtaining biological samples from patients with suspected malignant neoplasms of the epithelium and from a control group of healthy subjects. Next, a new marker of epithelial cancers that forms on the surface of cancer cells of epithelial origin is isolated. If the level of expression of the epithelial cancer marker in the samples obtained from the patients exceeds the level of expression of the marker in the samples obtained from healthy subjects, then the result indicates a high probability that epithelial cancer is present. The cancer antigen is a family of N-glycoproteins that have identical N-glycosylation and have a molecular mass of 55-85 kD. The detection method includes a capture reagent for the indicated cancer antigen, a detection reagent and a detection reagent.

EP 4 141 445 A1

**Description**

TECHNICAL FIELD OF THE INVENTION

The present invention relates to the fields of biotechnology and medicine, in particular to the field of cancer diagnostics, and describes a method of obtaining, isolating and a method of detecting a marker for epithelial carcinomas, which can be used for early diagnosis of malignant neoplasms of epithelial carcinogenesis. Specifically, the invention relates to the use of genetic and/or protein markers for the detection of cancer, and even more particularly for the use of genetic and/or protein markers for the diagnosis of epithelial cancers, in particular, the detection of various breast, prostate, and gastric carcinomas, lung carcinoma, ovarian carcinoma, and colon carcinoma among others.

The invention includes methods for obtaining a new tumor-specific antigen for detecting malignant tumors of epithelial tissues, describes a method of production, a method of isolation and a method of detecting a marker for epithelial carcinomas, comprising a polysaccharide fragment produced on the surface of malignant epithelial cancer cells due to special treatment of the biological material to be used for detecting malignant diseases of epithelial origin. According to the isolation method, a new marker for epithelial carcinomas, is formed when biological fluids such as blood or saliva from patients or tumor extracts are consistently exposed to specific heating treatment and used in a low pH medium. The invention also relates to methods of diagnosis and to the corresponding test kits, and describes a method of detecting a biomarker.

SUMMARY OF THE INVENTION

The specified cancer antigen comprises a family of N-glycoproteins with identical N-glycosylation, with a molecular weight of 55-85 kDa, which is formed upon conformational changes in the serum of patients or on the membrane surface of cancer epithelial cells due to the heating treatment in the range of 50-65 °C and the use of low pH value in the range of pH 2.5-5.0. Neoplastic cells not only produce but also take up alpha-fetoprotein (AFP) by means of surface glycoproteins with M.W. 55-85 kDa. In its native form, a glycoprotein with M.W. 55-85 kDa is not a differentiation antigen of epithelial tumors, and acquires its unique ability to differentiate healthy epithelial cells from tumor cells of epithelial tissue that had been transformed only within the process of chemical transformations resulting from or caused by the treatments described in this invention. Unlike hematological malignancies, epithelial tumor tissues in the process of malignant transformation gradually lose their differentiation due to the obliteration of the connections with the tumor microenvironment, which leads to varying degrees of cell differentiation loss, up to the epithelial-mesenchymal transition with the formation of the tumor stem cells and re-expression of the embryonic antigens. Epithelial tumor cells secrete a soluble fraction of the glycoprotein with M.W. 55-85 kDa, which, as a result of the treatment described in this invention, allows to quantitatively measure the cancer antigen, which in turn can be used as a diagnostic method for early detection of epithelial carcinomas, screening of patients at risk and for treatment monitoring of epithelial cancer. Measurement of the cancer antigen's level can be carried out along with other instrumental diagnostic methods such as ultrasound, X-ray, MRI, etc.

LEVEL OF TECHNOLOGY

Cancer is a malignant disease that begins with cell mutations and leads to atypical malignant cells dividing in an uncontrolled way, invading nearby tissues and spreading to other parts of the body, affecting various tissues and organs and disrupting their normal functioning. Cancer is one of the leading causes of morbidity and mortality worldwide. According to the World Health Organization (WHO), about 40% of people will be diagnosed with cancer at some point in their lives, and about 40% of those who become sick will die from the disease.

One of the main reasons for the lack of effectiveness of the currently existing cancer treatments is that most cancers are detected at advanced stages, when the survival rate is poor. Early detection of malignant neoplasms is the main factor determining the overall survival of patients with cancer, including epithelial cancers. When cancer is detected in a patient at stage I, the 5-year survival rate is about 90%, compared to 12% for patients diagnosed at stage IV, which is the advanced stage of the disease. Unlike common or metastatic forms of cancer, early stages of malignant neoplasms could be treated successfully. The main problem is the great difficulty in detecting early stages of cancer, since they progress without any symptoms and clinical expression. This leads to late detection of cancer pathologies and a delay in treatment, resulting in a poor clinical prognosis and causing high mortality in patients.

In relation to the above-mentioned situation, the World Health Organization (WHO) and the International Organization for Cancer Control (IARC) have made early detection of cancer a priority for doctors and scientists around the world. Currently, one of the methods for early detection of various cancer is the detection of specific biomolecules, the so-called "cancer markers", in biological samples, including blood, saliva, bone marrow tissue or tumor tissue formed during the neoplasm development. The identification of cancer markers in a patient's bodily fluids can provide a valuable approach to early diagnosis leading to timely cancer treatment and, as a result, a better prognosis. Besides early cancer detection, tumor-associated markers can be used as analytical tools for monitoring disease progression, detection of relapse, and for monitoring the effectiveness of chemotherapy, radiotherapy, or surgery. However, most of the currently known cancer markers are tissue-specific and, at the same time, do not possess high sensitivity in detecting the earliest stages of cancer.

As an example, the most commonly used markers of breast cancer, CA 15-3, prostate cancer PSA, stomach cancer CA 19-9, CA 72-4 and Carcino-Embryonic Antigen (CEA) are detectable in only 20 to 60% of tumors, with only 5 to 20% in early stages of cancer.

Thus, existing cancer markers fail to detect many cases of malignant neoplasms, especially at the early stages of their development, which leads to a large number of false-negative results that do not allow timely cancer detection and consequently prevent starting an effective treatment.

A novel cancer antigen associated with epithelial tumors, N-glycoprotein, can be generated and detected to varying degrees in the tissues of these tumors, as well as in various human biological fluids (including blood and saliva) according to the method described in this invention. Identification of the marker for epithelial carcinomas can be used independently as a unique marker for detecting malignant neoplasms of epithelial genesis, or in combination with other known cancer markers to create new diagnostic tools for early diagnosis of epithelial cancers.

This invention provides a novel method of detecting tumors of epithelial origin, including but not limited to breast carcinoma, prostate carcinoma, colon carcinoma, lung carcinoma and ovarian carcinoma, using the described cancer antigen, a carcinoma-specific marker.

The essence of this invention is the identification and isolation of a new cancer antigen - the marker for epithelial carcinomas, using a method that includes heating treatment and an acidic treatment - in the blood of asymptomatic cancer patients, and patients with locally advanced and metastatic forms of cancer, which will allow detecting and/or measuring an increased level of expression at the very beginning of tumor growth and revealing the onset of cancerogenesis in the human body long before any symptoms of cancer presence occur.

A method of isolating cancer antigen, the marker for epithelial carcinomas, includes: (a) obtaining samples of blood, tumor tissue, cancer cell lines, bone marrow tissue, saliva from patients with suspected malignant neoplasms of epithelial origin; (b) the use of thermal treatment in the range of 50 to 65 °C of the patient's biological fluid samples (blood, serum, saliva) or suspensions of tumor cells; (c) the use of an acidic medium with a low pH value in the range from 2 to 5. Preliminary studies showed that the specified cancer antigen is formed when patient's biological fluids (blood, serum or saliva) or tumor extracts are successively subjected to special heating treatment and the use of an acidic medium treatment. In its native form, the glycoprotein with M.W. 55-85 kDa is not a differentiation antigen of epithelial tumors, and acquires its unique ability to differentiate healthy epithelial cells from tumor cells of epithelial tissue that had been transformed only within the process of chemical transformations resulting from (or caused by the treatment) described in this invention. The studied blood serum samples from patients with cancer and blood serum samples from the control group of healthy people without malignant tumors did not show any differences before using the method specified in this invention. The difference between samples from healthy people and cancer patients becomes apparent only after the treatment described in this invention. According to the method of isolating cancer antigen, the initial protein complex undergoes hydrolysis, in which the conformational changes in proteins and the destruction of intermolecular bonds lead to the formation of an active polysaccharide antigenic determinant, which is in fact a new cancer antigen. The cancer antigen specified in the invention contains an N-Glycan with several branched fucosylated antennas and is linked to the protein part of the molecule by a hydrocarbon bond through the aminoacid asparagine.

A method of isolating cancer antigen, the marker for epithelial carcinomas, with M.W. 55-85 kDa, includes: (a) obtaining a suspension of cancer cells; (b) processing the cell suspension and obtaining a cell extract; (c) isolation and purification of cancer antigen from a cell extract by immunoaffinity chromatography using native porcine alpha-fetoprotein. Our preliminary studies have shown that the use of recombinant human and/or porcine AFP has no specific binding to a said cancer antigen and therefore cannot be used to purify said cancer antigen using affinity immunochromatography.

For the past two decades, AFP has been considered as a promising carrier molecule for an anticancer targeted drug that could be used to specifically target a malignant tumor. Both native and recombinant AFP have been used as vectors for the delivery of various cytotoxic agents and plant toxins to tumor cells (1, 2). The presence of AFP receptors in malignant tumors allows targeting them with various AFP-complexes and different toxic substances. However, in another work, we have demonstrated that only native porcine AFP provides the specificity required for targeted anticancer drugs (3). An example is a drug named AnVIPII,A, an oral dosage form of which containing a composition of a non-covalent porcine AFP complex and an apoptosis-inducing agent atractyloside. The drug is intended for the specific targeted delivery of atractyloside to cancer cells through AFP receptors on their surface (4).

A method of detecting a cancer antigen to identify patients with epithelial cancer in comparison with a control group of healthy people consists in:

(a) Obtaining samples of blood, serum, tumor tissue, culture of cancer cells, bone marrow tissue, and saliva from patients with suspected malignant epithelial neoplasms;

(b) Obtaining samples of blood, serum or saliva from a control group of healthy people without malignant tumors;

(c) A method of obtaining a new marker for epithelial carcinomas formed on the surface of cancer cells of epithelial carcinogenesis;

(d) If the expression level of the epithelial cancer marker in the indicated samples in (a) exceeds the expression level of the epithelial cancer marker in (b), then the result means a high probability (likelihood) of the presence of epithelial cancer.

In this case, a set of reagents for the detection of epithelial cancers contains: a) a reagent for capturing said cancer antigen, which may be an oligonucleotide or an antibody specific for a particular sequence of N-polysaccharides of said cancer antigen, the protein itself or a protein peptide; b) a detection reagent capable of detecting said cancer antigen associated with said capture reagent upon interaction with said cancer antigen; c) a detection reagent capable of detecting said cancer antigen associated with said capture reagent may be a luminescent, bioluminescent, enzymatic, fluorescent, or radioactive label attached to the isolated antigen.

DESCRIPTION OF THE INVENTION

The details of this invention describe methods for producing and purifying a novel cancer antigen, the marker for epithelial carcinomas, formed on the surface of epithelial cancer cells as a result of the described method of treatment, which can provide early detection of cancer, as well as reduce the number of false positive and false negative measurements.

The invention also relates to a method for determining the presence and/or concentration of a cancer antigen, for early detection of cancer, by performing any type of immunoassay (direct or competitive antigen-antibody binding, or a sandwich assay using an antibody having immunological reactivity against said antigen, and can be used in medicine.

The invention also relates to a set of reagents for cancer detection, comprising said cancer antigen and antibodies against said antigen.

Proteins that are secreted by cancer cells or are located on the membrane surface of malignant cells, independently or in combination with each other, can be used as markers for serum, tumor tissue, bone marrow tissue, or body fluid for detecting cancer, or as markers for monitoring the progression of an established malignant disease. The detection of such protein markers can be accomplished using methods known in the art and includes the use of monoclonal antibodies, polyclonal antisera, and the like. However, most of the currently known tumor markers are tissue-specific and, at the same time, do not possess high sensitivity in detecting the early stages of cancer. The results of published clinical studies using widely used tumor markers show that they are not highly sensitive and leave out many cases of malignant neoplasms, especially in the early stages of their development, which leads to a large number of false-negative results that do not allow timely detection of cancer and starting effective treatment.

As opposed to widespread cancer markers, a new cancer antigen associated with epithelial tissue tumors forms at the very beginning of cancerogenesis as a result of the treatment method described in this invention. It is formed to varying degrees on the membrane surface of these tumors, and also enters the bloodstream of cancer patients. It allows the described cancer antigen to be used as a unique marker for detecting malignant neoplasms of epithelial genesis at an early stage of the epithelial cancer development.

In particular, the present invention provides a method for detection of the presence and / or concentration of a cancer antigen, the marker for epithelial carcinomas, comprising:

(i) providing a biological sample;
(ii) treatment of the patient's biological fluids (blood, saliva) or tumor extracts by successive thermal exposure in the temperature range from 50 to 65 °C and using an acidic medium with a low pH value;
(iii) determining the level of expression of a protein marker for epithelial cancers in the specified sample.

In one aspect, overexpression of the protein marker for epithelial carcinomas in a patient is an indication that the patient is likely to have epithelial cancer.

To determine the level of expression of a cancer antigen characteristic for epithelial cancers, any suitable detection method can be used, including enzyme immunoassay, immunochemiluminescence assay, fluorescence assay, or immunochemical assay using a characteristic N-glycoside sequence of polysaccharides, or complementary at most portions of the sequence of polysaccharides of said cancer antigen, determining the level of cancer antigen, for example, using an antibody directed against said cancer antigen for epithelial carcinomas. Any suitable antibody can be used to determine the concentration of said cancer antigen, which can be a monoclonal antibody or a polyclonal antiserum. The method for determining the concentration of a cancer antigen can be carried out using any type of immunoassay (direct or competitive binding, or sandwich assay), or using an antibody chip. The present invention also provides a set of reagents for detecting a cancer antigen, comprising: a capture reagent of said cancer antigen and a detection reagent associated with a support capable of detecting said cancer antigen associated with said capture reagent.

The capture reagent of said cancer antigen may be an oligopeptide or antibody specific for the N-glycan sequence of the polysaccharides of said cancer antigen, the protein itself, or a protein peptide.

A detection reagent capable of detecting said cancer antigen associated with said capture reagent may be a luminescent, bioluminescent, enzymatic, fluorescent, or radioactive label attached to the isolated antigen.

This invention describes a method for the early detection of epithelial cancers based on the quantitative determination of the concentration of a cancer antigen specific to epithelial cancers, including:

(a) Obtaining samples of blood, tumor tissue, bone marrow tissue, saliva or urine from patients with suspected malignant neoplasms of the epithelium;

(b) Treatment of the patient's biological fluids (blood or saliva) or tumor extracts by sequentially thermal treatment in the temperature range from 50 to 65 ° C and using an acidic medium with a low pH value;

(c) Measuring the level of expression of the specified cancer antigen in the test sample; and

(d) Comparing the level of expression of the cancer antigen, the marker for epithelial carcinomas, in the indicated samples with the level of its expression in the control healthy samples.

Another aspect of the present invention may be its use as a screening tool for detecting epithelial cancers, including:

(a) Obtaining samples of blood, serum, tumor tissue, bone marrow tissue, saliva from patients with suspected malignant epithelial neoplasms;

(b) Treatment of the patient's biological fluids (blood, serum or saliva) or tumor extracts by sequentially thermal treatment in the temperature range from 50 to 65 ° C and using an acidic medium with a low pH value;

(c) Measuring the level of expression of the specified cancer antigen in the test sample;

(d) Comparing the level of expression of a cancer antigen, a marker for epithelial carcinomas, in the indicated samples with the level of expression in control healthy samples;

(e) In agreement with the method, any suitable detection method can be used, including an enzyme immunoassay, a chemiluminescence immunoassay, a fluorescence immunoassay, a radiometric immunoassay, or an immunochemical assay;

Another aspect of this invention may be its use to monitor the treatment of an already identified epithelial cancer, including:

(a) Obtaining the patient's biological samples of blood, serum, tumor tissue, bone marrow tissue, saliva or urine from patients with suspected malignant neoplasms of the epithelium;

(b) Treatment of the patient's biological fluids (blood, serum, urine, saliva) or tumor extracts by successive thermal treatment in the temperature range from 50 to 65 ° C and using an acidic medium with a low pH value;

(c) Measuring the initial level of expression of the specified cancer antigen in the test sample before the start of treatment; and

(d) Measuring the level of expression of the specified cancer antigen in the test samples of the patient during his treatment;

(e) Comparing the expression levels of a cancer antigen, the marker for epithelial carcinomas, in the indicated samples during treatment with the initial level of its expression prior to treatment;

(f) In agreement with the method, any suitable detection method can be used, including an enzyme immunoassay, an chemiluminescence immunoassay, a fluorescence immunoassay, a radiometric immunoassay, or an immunochemical assay;

(g) The test sample can be obtained from blood, serum, bone marrow tissue, or saliva.

PURPOSE OF THE INVENTION

The main purpose of the present invention is to provide a method for identifying, isolating and detecting a novel cancer antigen that includes a family of N-glycoproteins with identical N-glycosylation, with a molecular weight in the range 55-85 kDa, associated with epithelial tumors, for the detection and screening of carcinomas. In particular, the cancer antigen is associated with breast carcinoma, prostate carcinoma, gastric carcinoma, lung carcinoma, ovarian carcinoma, and colon carcinoma.

BRIEF DESCRIPTION OF DRAWINGS

1. Figure 1

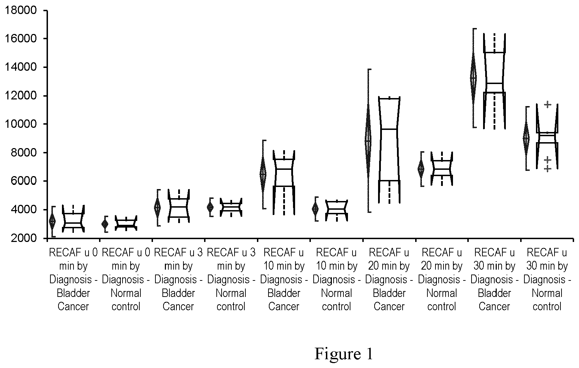

Figure 1 shows the effect of heating time on serum samples for discrimination between healthy controls and patients with bladder cancer.

2. Figure 2

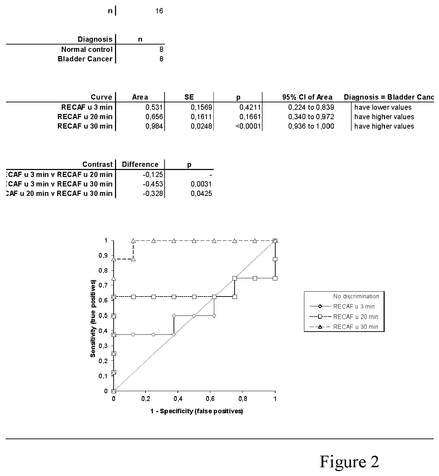

Figure 2 shows the characteristic ROC curves for the studied bladder cancer samples and healthy controls, in depends on the time of their heating using the described cancer antigen.

3. Figure 3

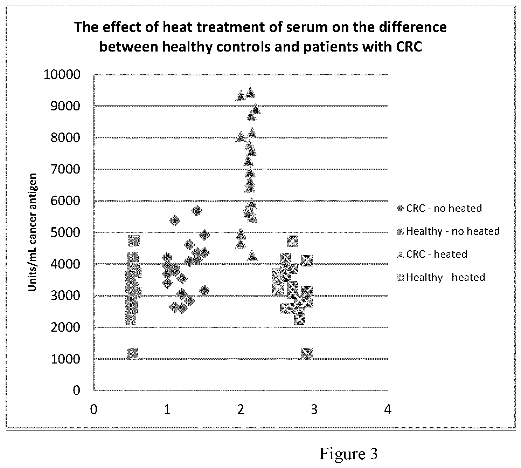

Figure 3 shows the effect of the heat treatment of serum samples on the difference between healthy controls and patients with colorectal cancer (CRC).

4. Figure 4

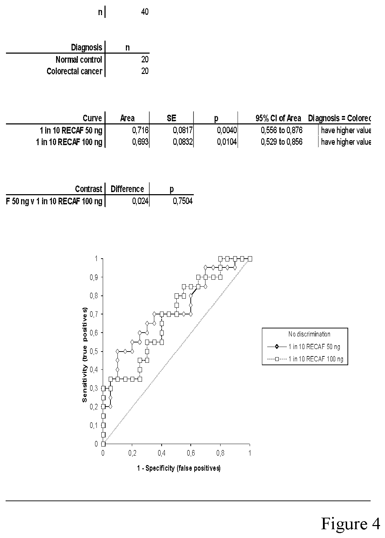

Figure 4 shows the characteristic ROC curves for the studied samples of colorectal cancer and healthy controls without prior heating treatment of serum using different concentrations of the described cancer antigen.

5. Figure 5

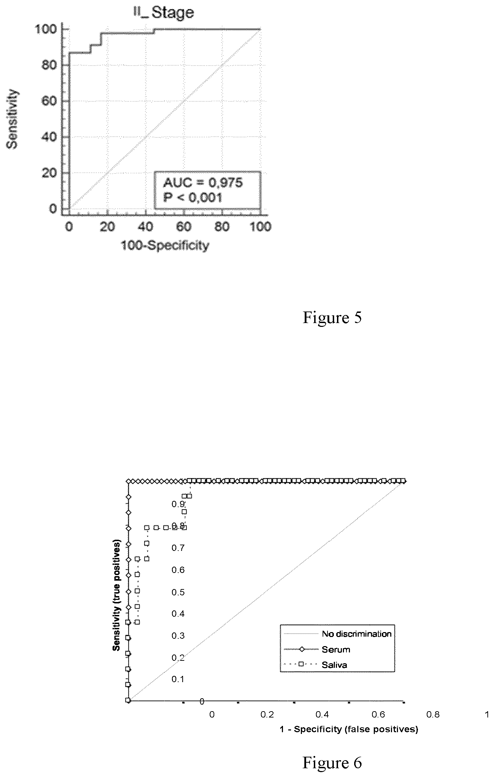

Figure 5 shows the characteristic ROC-curve for the studied samples of colorectal cancer and healthy controls with thermally pre-heated serum using the described cancer antigen.

6. Figure 6
Figure 6 shows the characteristic ROC curve of paired serum and saliva samples for 14 breast cancer patients and 58 healthy controls.

7. Figure 7 shows polyacrylamide gel electrophoresis and Western blotting, which show glycoprotein with a molecular weight of 55-85 kDa, isolated in pure form from a suspension of human chronic myelogenous leukemia K-562 cancer cells, recognized by specific monoclonal IgMEC antibodies against cancer antigen.

8. Figure 8

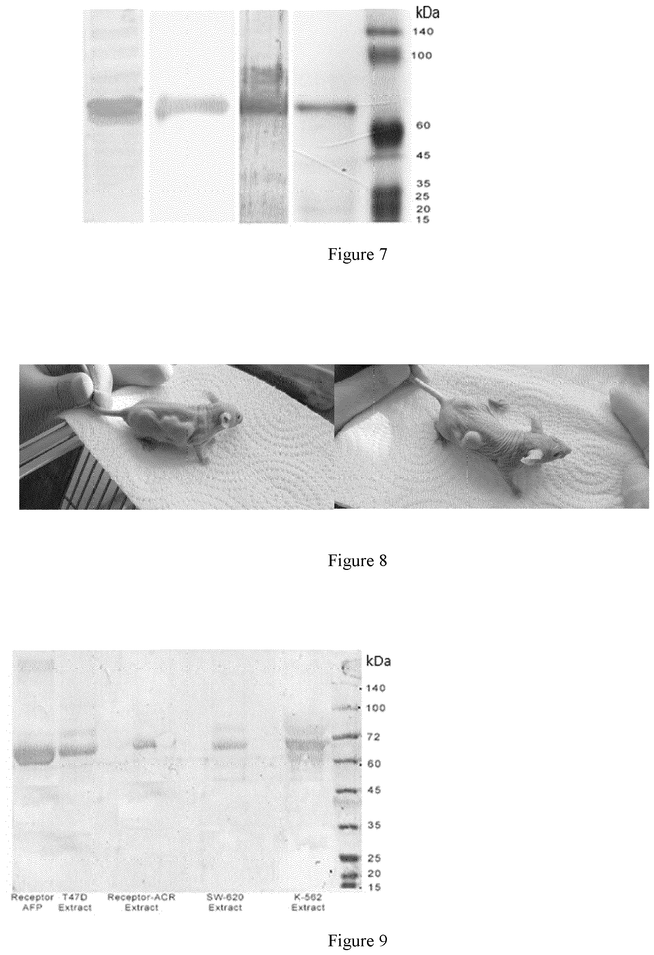

Figure 8 shows experimental Balb / c mice with human subcutaneous xenografts of human tumors that were used in the study to obtain tumor cell extracts.

9. Figure 9
Figure 9 shows Western blotting of suspensions of various tumor homogenates (extracts of homogenized cells) with IgMEC monoclonal antibodies against said cancer antigen 60-65 kDa and goat antibodies against mouse IgM-HRP.

10. Figure 10

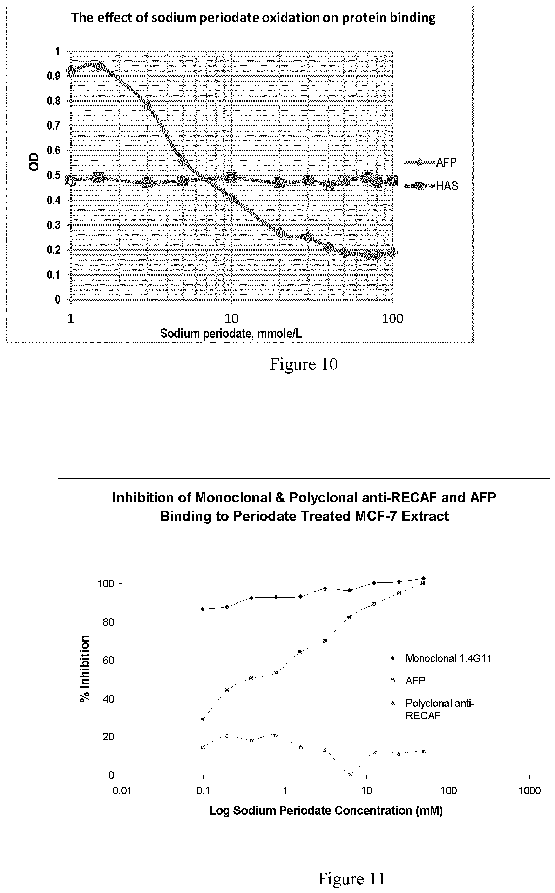

Figure 10 shows the effect of periodate oxidation on protein binding. Periodate destroys the structure of sugars.

11. Figure 11
Figure 11 shows the inhibition curves for IgMEC and AFP after serial dilutions of periodate on a sorbed extract of MCF-7 breast adenocarcinoma cancer cells.

12. Figure 12

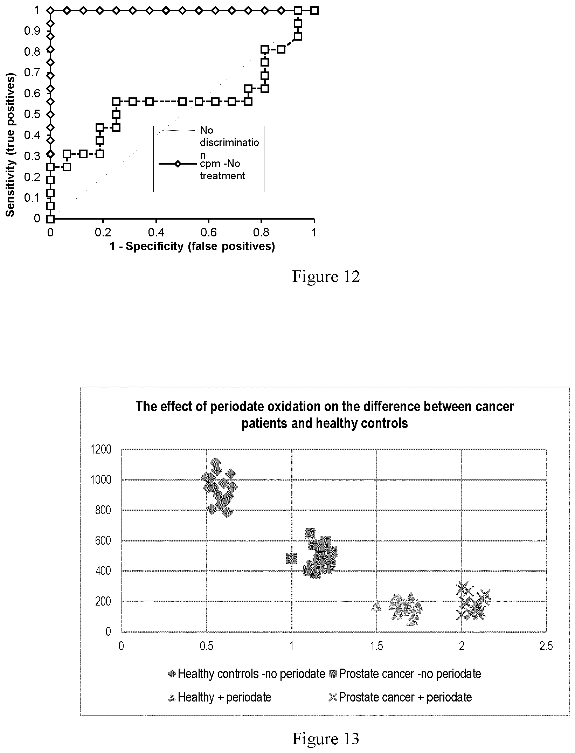

Figure 12 shows the ROC characteristic curve of samples subjected to periodate oxidation compared to untreated control serum samples.

13. Figure 13
Figure 13 shows the effect of periodate oxidation on the difference between patients and healthy controls.

14. Figure 14

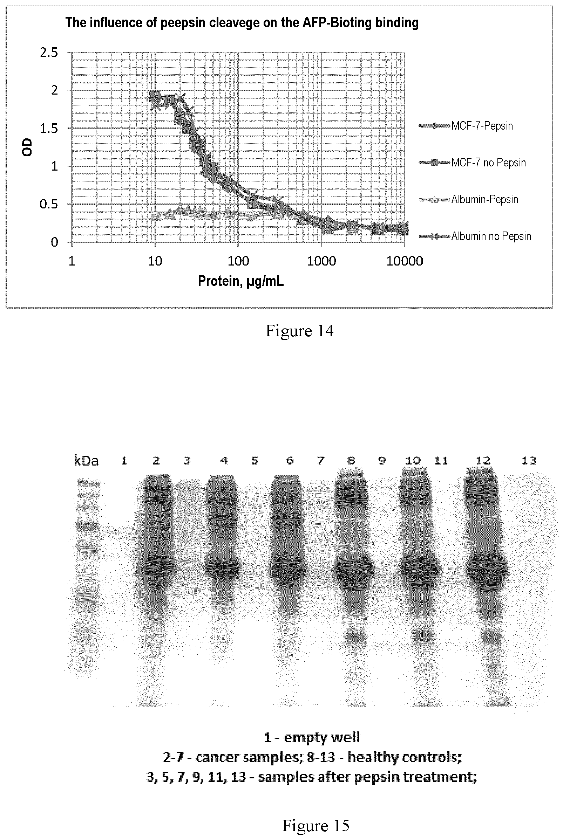

Figure 14 shows the inhibition curves of the binding of AFP-Biotin to pepsin-cleaved proteins.

15. Figure 15
Figure 15 shows polyacrylamide gel electrophoresis of serum samples before and after pepsin digestion.

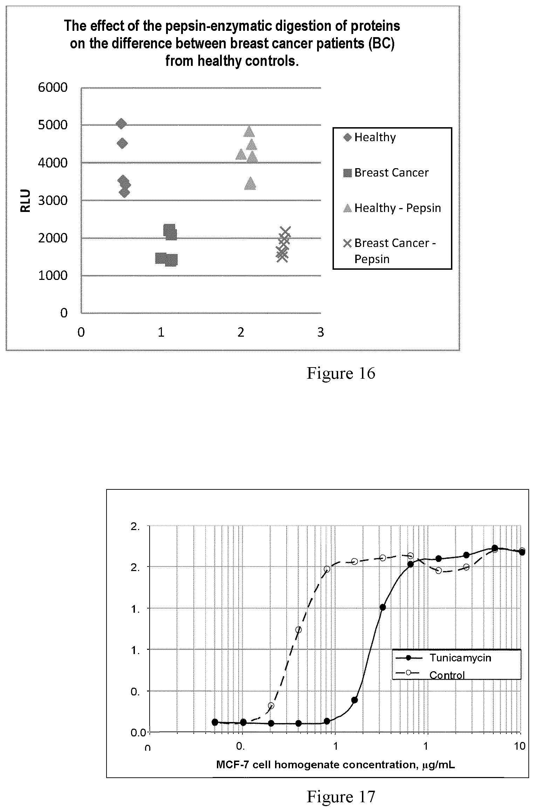

16. Figure 16 shows the effect of enzymatic digestion of proteins by pepsin on the difference between breast cancer patients (BC) from healthy controls.

17. Figure 17
Figure 17 shows the binding of AFP to MCF-7 homogenates from cells treated or not treated with Tunicamycin, a specific N-glycosylation inhibitor.

18. Figure 18

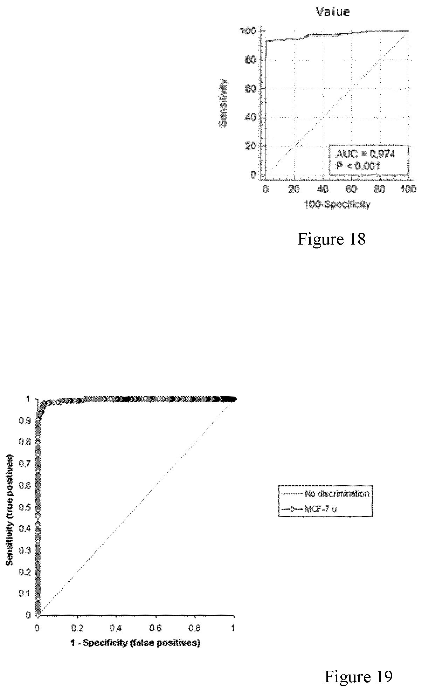

Figure 18 shows the ROC characteristic curve for the tested clinical samples.

19. Figure 19
Figure 19 shows the ROC characteristic curve for the investigated set of samples (N = 500).

20. Figure 20

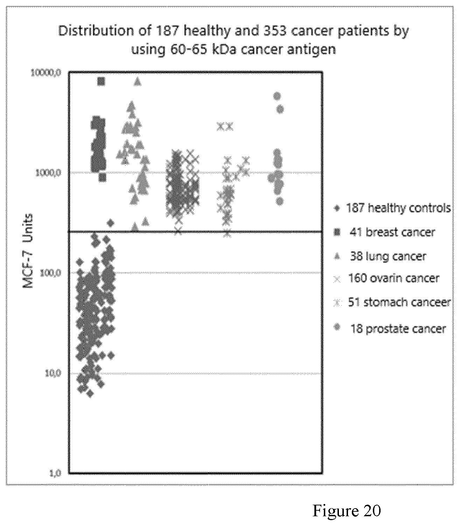

Figure 20 shows the distribution of a panel of test samples (patients with various carcinomas and healthy controls) using a cancer antigen.

DETAILED DESCRIPTION OF THE INVENTION

The invention relates to the field of biotechnology and medicine, specifically to a method for detection the presence and/or concentration of a marker for epithelial carcinomas, in a sample, for early detection of cancer, a method for its quantification, a method for its isolation, in which the antigen to be detected can be found in a state associated with alpha-fetoprotein on the membrane surface of epithelial tumor cells, and is activated only by heat treatment and acidic pH. The novel tumor-associated antigen comprises of a family of N-glycoproteins with identical N-glycosylation, with a molecular weight of 55-85 kDa, which contains an Asn-GlcNAc N-glycosidic bond and several external highly branched polysaccharide antennas. This marker protein is formed in large quantities on the membrane surface of the malignant epithelial cells at the very beginning of carcinogenesis, which makes it a specific marker associated with epithelial carcinomas. Epithelial tumors of the prostate, breast or ovary develop from the superficial or glandular epithelium, are distinguished by the appearance of atypical epithelial cells, their high proliferation, which can progress and degenerate into cell carcinoma. The process of formation of a cancer antigen associated with cancer epithelial cells begins with malignant transformation of the epithelium during the epithelial-mesenchymal transition and the formation of uniform cancer stem cells from a variety of epithelial cells with different morphological and epigenetic characteristics, on the membrane of which a complex of cancer antigen with alpha-fetoprotein is formed, which is required for cellular nutrition. This invention describes a method for activating and isolating of the cancer antigen for its use in cancer detection. Before describing the ways of applying the present invention, we shall introduce some definitions of terms that will be used in the text.

The term "cancer antigen" or "tumor-associated antigen" means a gene, gene fragment, RNA, RNA fragment, protein, glycoprotein or glycoprotein fragment associated with or identifying a molecule associated with a malignant neoplasm of epithelial origin. The specified cancer antigen as part of the present invention does not include molecules that were previously known and are associated with various types of epithelial cancer, in particular, breast cancer (cancer antigen CA-15.3), prostate cancer (PSA), stomach cancer (cancer antigen CA19-9 and CA74-2, CEA). However, the cancer antigen of this invention can be used in new and innovative combinations with previously known cancer antigens for specific cancers.

The term "marker" refers to a molecule that is qualitatively or quantitatively associated with the presence of a biological phenomenon, for example, the presence of transformed epithelial cells on the surface of malignant tumors. Examples of "markers" include polynucleotides such as a gene or gene fragment, RNA or RNA fragment, or a gene product including polypeptides such as peptides, oligopeptides, proteins, glycoproteins or glycoprotein fragments; or any identifying molecule, such as antibodies or antibody fragments, linked directly to a marker or through a mechanism that determines a biological phenomenon.

The marker of the present invention includes a nucleotide sequence, in particular a sequence of the entire length of a protein, any coding sequences, any fragments, a polysaccharide sequence linked to a protein as described above.

As indicated in this invention, the term "antibodies" and similar terms refers to molecules of immunoglobulins and immunologically active portions of immunoglobulin (Ig) molecules, i. E. molecules that contain an antigen-binding fragment, which specifically binds to various functional groups of the antigen through aromatic side chains of short hydrophilic residues, which underlies the affinity and specificity of recognition of the antibody-antigen complex through the accumulation of a set of relatively weak non-covalent interactions. This includes, but is not limited to monoclonal, polyclonal, chimeric, single chain antibody, Fc, Fab, Fab' and Fab2 fragments, as well as the expression of Fab fragments. Antibody molecules belong to any class such as IgG, IgM, IgA, IgE and IgD, which differ from each other in the nature of the heavy chain present in the molecule. They also include subclasses such as IgGl, IgG2 and others as well as nanobodies The light chain can be a kappa chain or a lambda chain. Hereinafter, reference to antibodies includes reference to all classes, subclasses, and types of antibodies. The class of antibodies also includes chimeric antibodies, for example, monoclonal antibodies or fragments thereof that are specific for more than one source, for example, a sequence of a mouse, rabbit, goat or human.

The term "cancer" or "cancerous" describes a physiological condition in mammals that is usually characterized by uncontrolled proliferation of tumor cells. Cancer and oncological pathology can be associated, for example, with metastases, can interfere with the normal functioning of the environment of tumor cells, can release an increased level of cytokines or other products of secretion of tumor cells, suppress or, conversely, provoke inflammatory processes or the body's immune response, neoplasia, oncogenesis and precancerous conditions, malignant neoplasms, invasive lesions of the tumor environment or distant organs such as lymph nodes, etc. Melanomas, which also belong to the term "cancer", deserve special attention.

The term "tumor" refers to the growth and proliferation of neoplastic cells, whether they are malignant or benign, and to all atypical cells exhibiting pronounced nuclear polymorphism and tumor cells and tissues.

The term "epithelial cancer" refers to a type of malignant tumor that develops from cells of the epithelial tissue of various organs (mucous membranes, skin, many internal organs). This type of tumor is called carcinoma. These types of tumors have the ability to metastasize.

The term "expression" includes the production of glycoproteins, polynucleotides or polypeptides, in particular, the production of RNA from a gene or portion of a gene, and includes the production of a polypeptide encoded by an RNA or a gene, and the appearance of a detectable antigen associated with expression. For example, complex formation by polypeptide-polypeptide interaction or polypeptide-nucleotide interaction or the like is within the scope of the term "expression". Another example is the generation, by the method of processing of the present invention, of a quantitatively detectable cancer antigen associated with malignant tumors of epithelial tissues.

The term "threshold" refers to the level of a specified cancer antigen, beyond which a glycoprotein, oligopeptide or polypeptide serves as a marker for the presence of a malignant disease in a patient. The threshold will depend on the established diagnostic model derived experimentally from pilot clinical trials such as those described in the Examples below. Depending on the diagnostic model used, the threshold value can be set to achieve maximum sensitivity or maximum specificity or minimum error (maximum degree of classification). For example, a higher threshold can be set to achieve minimal errors, but this can lead to lower sensitivity. Therefore, for any given diagnostic model, clinical studies will be used to establish the expression threshold that usually achieves the highest sensitivity with the lowest error rate.

The term "susceptibility" refers to the proportion of people with a disease who test positive (by model). Thus, increased sensitivity means fewer false negative test results.

The term "specificity" refers to the proportion of individuals without disease who have a (model) test negative. Thus, increased specificity means fewer false positive test results.

The term "microchip" refers to the ordered or disordered arrangement of capture agents, preferably polynucleotides (e.g., probes) or polypeptides on a support.

The term "glycoprotein" refers to compounds in the molecules of which oligo- or polysaccharide residues are covalently O- or N-glycosidic bonds to polypeptide chains of the protein. Hydroxyamino acids are involved in the formation of O-glycosidic bonds. Combinations of galactose (Ga1) -hydroxylysine, galactose or arabinose hydroxyproline, serine or threonine are known. The addition of N-acetylglucosamine (GlcNAc), mannose (Man) and galactose residues to serine, fucose (Fuc) to threonine is known, but the vast majority of such glycoproteins contains carbohydrate-protein bond is the same disaccharide fragment, the so-called rod, which consists of galactose and N-acetylgalactosamine (GalNAc) residues and has the structure Gaipi-3GalNAcal. In glycoproteins in which the carbohydrate and polypeptide chains are linked by N-glycosidic (glycosyl-amide) bonds, the N-acetylglucosamine residue is linked to the N atom of the amide group of the asparagine residue in the polypeptide chain.

The term "oligonucleotide" refers to a polynucleotide, usually a probe or primer, including, but not limited to, single-stranded deoxyribonucleotides, single-stranded or double-stranded ribonucleotides, RNA: DNA hybrids, and double-stranded DNA. Oligonucleotides, such as single-stranded DNA probe oligonucleotides, are often synthesized by chemical methods, for example, using automatic oligonucleotide synthesizers that are commercially available, or by various other methods, including in vitro expression systems, recombinant methods, and expression in cells and organisms.

"Polypeptide" in the context of the present description refers to the sequence of an oligopeptide, peptide or glycoprotein, or a fragment thereof, as well as natural, recombinant, synthetic or semi-synthetic molecules. When the term "polypeptide" is used herein to refer to an amino acid sequence of a naturally occurring protein molecule, "polypeptide" and the like are not intended to limit the amino acid sequence to the entire native amino acid sequence to describe the full length of the molecule. It should be understood that each reference to a "polypeptide" or similar term in this document will include the entire sequence, as well as any fragments, derivatives or variations thereof.

The practice of the present invention will be based on conventional techniques in molecular biology (including recombinant techniques), microbiology, cell biology, and biochemistry, which are known to those skilled in the art. These methods are fully described in the literature, such as (5, 6).

## DESCRIPTION OF EMBODIMENTS

Usually, tumor markers are differentially expressed in tumor tissue and in corresponding healthy tissue, which makes it possible to distinguish cancer patients from healthy people. Nevertheless, it is likely that the physiological and morphological characteristics of tumor tissues can lead to differences in accumulation of markers in serum and other biological fluids, even in the absence of their overexpression in tumor tissue. In particular, it can be assumed that the abnormal polarity of tumor cells, a high degree of tumor vascularization and high intratumoral pressure contribute to the outflow of specific markers from tumor tissue as compared to non-malignant tissue. Therefore, it is believed that a secreted cancer antigen or glycoprotein that is abundantly expressed in tumor tissues but not expressed in healthy epithelial tissue would be a useful marker for epithelial carcinomas.

## IMMUNOGISTOCHEMISTRY AND PROTEOMICS

Immunohistochemical methods are also suitable for determination of the expression levels of the proliferative markers in accordance with this invention. Thus, antibodies or antisera, preferably polyclonal antisera and most preferably monoclonal antibodies specific for said antigen, are used to detect its expression. Antibodies can be detected by directly labeling the antibodies themselves, for example, with radioactive labels, fluorescent labels, luminescent and bioluminescent labels, hapten labels, such as biotin, or an enzyme labels, such as horseradish peroxidase or alkaline phosphatase. Alternatively, an unlabeled primary antibody is used in combination with a labeled secondary antibody containing an antiserum, a polyclonal antiserum, or a monoclonal antibody specific for the primary antibody. Immunohistochemistry protocols and kits are well known in the art and are commercially available.

Proteomics can be used to analyze polypeptides presence in a sample (e.g., serum, tissue, organism, or cell culture) at a given point in time. In particular, proteomic methods can be used to assess global changes in the expression of a polypeptide in a sample (also called expression proteomics). Proteomic analysis usually includes:

(1) separation of individual polypeptides in a sample by 2-D gel electrophoresis (2-D PAGE);

(2) identifying individual polypeptides recovered from the gel, for example, by mass spectrometry or N-terminal sequencing, and

(3) data analysis using bioinformatics. Proteomic techniques are valuable additions to other gene expression profiling techniques and can be used alone or in combination with other techniques to detect proliferation marker products of the present invention. Application of magnetic particles

Application of magnetic nanoparticles in fluorescent immunoassays to activate flow cytometry (7). A variation of hybridization technology is the QuantiGene Plexe assay (Genospectra, Fremont), which combines fluorescent bead support with DNA branched signal amplification. Another option for hybridization technology is Quantikine® mRNA analysis (R&D Systems, Minneapolis). The method is as described in the manufacturer's instructions. Briefly, the assay uses oligonucleotide hybridization probes conjugated to digoxigenin. Hybridization is detected using antibodies against digoxigenin coupled to alkaline phosphatase in colorimetric assays. Other methods are well known in the art and require no further description herein.

Enzyme-linked immunoassays (ELISA)

Briefly, in sandwich ELISA assays, a polyclonal or monoclonal antibody against a specified antigen binds to a solid support (8, 9), suspension or magnetic nanoparticles. Other methods are well known in the art and require no further description herein. Monoclonal antibodies can be obtained from hybridoma or isolated from the phage antibody libraries. Nonspecific binding sites are blocked by non-target protein drugs and surfactants or detergents. The capture antibody is then incubated with a patient's serum or tissue sample containing said antigen. The mixture is washed prior to incubation of the antibody-antigen complex with a second antibody that detects said antigen. The second antibody is usually conjugated to a peroxidase tag or a fluorescent or luminescent molecule, or other labeled molecule that can be found in an enzymatic reaction, or to a third antibody conjugated to the tag. Alternatively, in direct ELISA methods, a preparation containing said cancer antigen can be bound to a support or bead, and the target antigen can be detected directly using an antibody-antigen conjugate. Methods of obtaining monoclonal antibodies and polyclonal antisera are well known in the art and require no further description herein.

Chemiluminescence immunoassay (CLIA)

Briefly, in one-step competitive variants of solid phase chemiluminescent immunoassay, a polyclonal or monoclonal antibody against a specified antigen binds to a solid support, suspension or magnetic nanoparticles. Other methods are known in the art and do not require further description herein. Monoclonal antibodies can be obtained from hybridoma or isolated from phage antibody libraries. Nonspecific binding sites are blocked by non-target protein drugs and surfactants or detergents. The capture antibody is then incubated with a mixture of a serum or tissue sample from a patient containing said antigen and a luminescent antigen conjugate for competitive binding. Unbound components are removed during washing, and the resulting antibody-antigen immuno-complex with a luminescent label, which detects the specified antigen, is read using a luminescent signal. The value of the registered luminescent signal of the substrate oxidation product is inversely proportional to the concentration of the specified cancer antigen in the analyzed sample.

Immunodetection.

The described methods can also be used for immunodetection of the specified antigen in the serum or tissue of patients with suspected epithelial cancer; for immunodetection of the specified antigen in the serum or tissue of patients with epithelial cancers, taken before and after surgical cancer resection; for immunodetection of the specified antigen in patients with epithelial cancers; before treatment and immunodetection of the specified antigen in the serum, saliva and bone marrow of patients with epithelial cancer, including breast cancer, prostate cancer, lung cancer, stomach cancer, bowel cancer, uterine cancer, ovarian cancer, and others. The specified antigen can be detected in serum samples or tissues using standard methods of immunodetection, such as immunoblotting or immunoprecipitation (9). In immunoblotting, protein preparations from tissue or liquid containing the specified antigen are subjected to SDS-gel electrophoresis in denaturing or native (non-denaturing) conditions. The proteins are then transferred to a membrane base, such as nitrocellulose or nylon. Then the specified antigen reacts directly or indirectly with monoclonal or polyclonal antibodies, as described in standard methods for immunohistochemistry. In another case, in some preparations, proteins can be applied directly to membranes without prior electrophoretic separation. The signal can be quantified using densitometry. In immunoprecipitation, a soluble preparation containing said antigen is incubated with monoclonal or polyclonal antibodies against said antigen. The reaction is then incubated with inert particles made from agarose or polyacrylamide with covalently attached protein A or protein G. Protein A or G beads specifically interact with antibodies to form an immobilized antibody-antigen-antigen complex bound to the particle. After washing, the bound said antigen can be detected and quantified by Western blotting or ELISA.

Determination of the cutoff value

For tests using the specified antigen, the cutoff values will be obtained that will allow the sample to be called positive or negative for the presence of epithelial cancer. These cutoff values will be determined by analyzing cohorts of patients being screened for epithelial cancers for each assay developed. Cutoff values can be different for various test applications. For example, cutoffs for using a test in a population-based screening will be determined using cohorts of patients who are conventionally healthy and asymptomatic of cancer, and these cutoffs may differ from those used in tests for patients already diagnosed with cancer, which are under doctor's surveillance. The cutoff can be chosen to provide a practical level of the test specificity in the required clinical setting. That means a specificity that provides reasonable sensitivity without an excessive number of false positives. This specificity can be in the range of 90-95%. An alternative method for obtaining a cutoff is to plot the sensitivity-to-specificity for different test thresholds (ROC curves) and then select the point of inflection of the curve.

As an alternative to specific cutoff values, the test ranges may be used, which provide a different degree of cancer likelihood and which have different clinical implications associated with them. For example, a test may have three cutoff ranges: one is associated with a high (e.g., 90%) risk of having epithelial cancer, the second one is associated with a low risk of epithelial cancer, and the third is associated with suspicion on cancer. For the grey zone range, recommendations can be defined for repeated testing within a specified time period.

Antibodies to antigen associated with epithelial cancer:
In further aspects, the present invention includes the production of antibodies against the specified antigen associated with epithelial cancer. Once a cancer antigen is isolated and identified, it can be produced in an amount sufficient to stimulate an immunological response. In some cases, a full-length specified antigen may be used, and in others, a peptide fragment of the specified antigen may be sufficient as an immunogen. The immunogen can be injected into a suitable host (e.g., mouse, rabbit, etc.) and, if necessary, an adjuvant, such as Freund's complete or incomplete adjuvant, can be administered to enhance the immune response. It is well known that the production of antibodies is routine in the field of immunology and needs no further description. As a result, antibodies, including monoclonal or phage display antibodies, can be produced against said antigen, identified using the methods described herein.

In other embodiments, antibodies can be produced against a protein or glycan fragment of a tumor marker, a cancer antigen identified herein, or against an oligonucleotide sequence unique to said marker. Although the disclosed protein is glycosylated, differences in glycosylation patterns may, under certain circumstances, lead to the misidentification of forms of said antigen that do not have conventional glycosylation patterns or have modifications in glycosylation.

In certain aspects of the invention, the immunogens of said antigen may comprise individual glycosylated fragments of said antigen or a characteristic N-glycosidic sequence of polysaccharides, attached or not attached to a hapten protein.

It is possible to get vectors containing antigen-encoding oligonucleotides. Many such vectors can be based on standard vectors known in the art. Vectors can be used to transfect a variety of cell lines to generate antigen-producing cell lines. Such cell lines can be used to produce the desired amounts of the specified antigen for the development of specific antibodies or other reagents for the specified antigen detection, or to standardize the developed assays for the specified antigen.

DESCRIPTION OF THE METHODS FOR PRACTICAL APPLICATION OF THE INVENTION

The invention describes a method of isolating a marker from epithelial carcinomas containing a polysaccharide fragment that is expressed on the surface of epithelial cancer cells, which can be used to detect cancer diseases of epithelial origin.

In one embodiment, the present invention relates to a method of production of a cancer antigen, the marker for epithelial carcinomas, and includes:

(a) Obtaining samples of blood, serum, tumor tissue, culture of cancer cells, bone marrow tissue, saliva or urine from patients with suspected malignant epithelial neoplasms;

(b) The use of thermal treatment in the heating range from 50 to 65 ° C of the patient's biological fluids (blood, serum, urine, saliva) or suspensions of tumor cells;

(c) The use of an acidic medium with a low pH value in the range 2.0 to 5.0. Preliminary studies have shown that the specified cancer antigen is formed only when the patient's biological fluids (blood, serum, urine, saliva) or tumor extracts are successively subjected to special heat treatment and the use of a medium with a low pH value.

In one embodiment, the present invention relates to a method for isolating a marker for epithelial carcinomas, the method comprising:

(a) Obtaining a cell extract suspension;

(b) Isolating and purifying said antigen from a cell extract suspension by immunoaffinity chromatography on a column with immobilized antibody;

(c) Determining the concentration and purity of the isolated cancer antigen.

For the purposes of the present invention, a suspension of K-562 cells, obtained from the pleural cavity of a patient with chronic myeloid leukemia in the terminal stage of the disease is used. The cell line can be obtained from commercial repositories or from new primary cancer cells obtained from the patient.

A new cancer antigen 55-85 kDa, associated with epithelial tumors from the very beginning of cancerogenesis, as a result of the treatment method described in this invention, is formed to varying degrees on the membrane surface of these tumors, and also enters the bloodstream of cancer patients, which makes it possible to use the described cancer antigen as a unique marker for detecting malignant neoplasms of epithelial genesis for use in early diagnosis of epithelial cancer.

In one embodiment, the cancer-associated antigen is detected using antibodies that specifically bind to said epithelial cancer-associated antigen. Antibodies against the cancer-associated antigen can be prepared using methods known in the art.

Antibodies that specifically react with said cancer antigen, or labeled derivatives thereof, can be used to detect a cancer-associated antigen in a variety of samples (e.g. biological materials). They can be used as diagnostic or prognostic reagents, and they can also be used to detect pathologies by means of the protein expression or pathologies of structure and/or pathologies associated with the tissue, cellular or subcellular location of the specified antigen associated with cancer. *In-vitro* diagnostic immunoassays can also be used to evaluate or monitor the effectiveness of specific treatment options.

The antibodies can be used in any of the known types of immunoassays based on the binding interaction of the antigenic determinant of said cancer associated antigen and specific antibodies. Examples of such assays are radioimmunoassays, enzyme immunoassays, immunofluorescence, immunoluminescence, immunochemiluminescence, immunoprecipitation, latex agglutination, hemagglutination, and histochemical tests. Antibodies can be used to detect and quantify a cancer-associated antigen in samples to reveal the role of said antigen in cancer and to detect early stages of cancer.

The antibody or sample can be immobilized (sorbed) onto a carrier or solid support capable of fixing cells, antibodies, etc. For example, the carrier material or solid support can be nitrocellulose, polystyrene, or glass, polyacrylamides, and magnetite. The carrier material can have any possible configuration, including spherical (e.g., beads), cylindrical (e.g., inner surface of a tube or well, or outer surface of a probe), or flat (e.g., microplate well, plate, test strip).

In one embodiment, the present invention provides a method for detecting cancer cells or monitoring cancer in an individual who has or is suspected of having cancer, the method comprising:

(a) Obtaining a sample from an individual;

(b) Treatment of the patient's biological fluids (blood, serum, urine, saliva) or tumor extracts by sequentially thermal exposure in the temperature range 50 to 65 ° C and using an acidic medium with a low pH value;

(c) Bringing the sample into contact with an antibody that binds to the specified antigen associated with epithelial cancer;

(d) Determining the level of cancer-associated antigen in the sample; and

(e) Comparing the level of a cancer-associated antigen in the sample with a control sample, where elevated levels of cancer-associated antigen compared to the control without disease indicate that the individual has cancer.

The term "sample" can refer to any blood serum sample. The term "control sample" can refer to any sample that is used to establish a baseline or normal level of a specified antigen and which is a blood serum sample from a patient.

In one embodiment, the invention relates to a method for monitoring cancer treatment in an individual, the method comprising:

(a) Obtaining a sample from an individual;

(b) Treatment of the patient's biological fluids (blood, serum, urine, saliva) or tumor extracts by sequentially thermal exposure in the 50 to 65 °C temperature range and using a medium with a low pH value;

(c) Determining the level of expression of a cancer-associated antigen in the sample;

(d) Repeating steps (a) and (b) at a later time point and comparing the result in step (b) with the result in step (c), where the difference in the level of expression of the cancer-associated antigen is indicative of the progression of cancer in the individual.

In particular, elevated levels of a cancer-associated antigen at a later point in time may indicate that the cancer is progressing and that treatment (if used) is not effective. In contrast, decreased levels of a cancer-associated antigen at a later time point may indicate that cancer relapses and that treatment (if used) is effective.

Treatment monitoring of cancer patients is an important problem and is necessary to assess the treatment efficacy and to make timely decisions on changing the course of chemotherapy. Currently, expensive instrumental methods of analysis are mainly used for cancer to monitoring, since there are no highly sensitive biomarkers that can correctly reflect the tumor response to treatment. Previous pilot clinical studies on patients with solid tumors of the stomach and intestines have shown that this antigen associated with epithelial malignant neoplasms can be used to assess the effectiveness of treatment.

Thus, the present invention can also be used to monitor treatment and control the progression of tumor growth, to control the occurrence of recurrent tumor growth, and to study the effectiveness of a particular treatment option. In particular, the method can be used to confirm the elimination of all tumor tissue after surgery, the effectiveness of the performed anticancer chemotherapy and/or radiation therapy.

The following examples illustrate the present invention:

Example 1: A method of generating and detecting a new cancer antigen in the serum of patients with bladder carcinoma and colon carcinoma.

All analyzed blood serum samples from patients with verified epithelial oncological disease underwent preliminary sample preparation, which consists in thermal heating of all samples in a water bath at a temperature of 50 to 65° C for 30 minutes at sample volumes of 0.5 - 1 ml. Heat treated serum samples were compared to similar untreated samples using competitive binding to a conjugate of purified cancer antigen and monoclonal antibodies against said cancer antigen using a competitive binding immunoassay. In a competitive variant of the solid-phase immunochemiluminescence assay, monoclonal antibodies against the indicated antigen were used, which was bound to a solid support of the plate wells. Nonspecific binding sites were blocked using a special acidic buffer solution based on dicarboxylic acids containing polysorbate, a nonionic surfactant. Then the capture antibody was incubated with a mixture of serum sample, containing the specified antigen, and a luminescent antigen conjugate for competitive binding. Unbound components were removed by washing, and the resulting antibody/antigen immuno-complex with a luminescent label, which detects the specified antigen, was read using a luminescence reader. The value of the registered luminescent signal of the substrate oxidation product is inversely proportional to the content of the specified cancer antigen in the analyzed sample. The results of these experiments are shown below in Table 1 and in Figures 1-5.

Table 1. Influence of heating time on the difference between blood serum samples from healthy people and sick patients.

| Diagnosis | CA, U/mL - 0 min | CA, U/mL - 3 min | CA, U/mL - 10 min | CA, U/mL - 20 min | CA, U/mL - 30 min |
|---|---|---|---|---|---|
| Sample 1 bladder cancer | 3821 | 4876 | 7329 | 7598 | 11928 |
| Sample 2 bladder cancer | 3102 | 3478 | 3678 | 5366 | 5366 |
| Sample 3 bladder cancer | 2790 | 4383 | 6349 | 6754 | 10115 |
| Sample 4 bladder cancer | 4279 | 5411 | 8119 | 8188 | 11887 |
| Sample 5 bladder cancer | 2457 | 3429 | 5558 | 5878 | 11469 |
| Sample 6 bladder cancer | 3377 | 4318 | 7530 | 9175 | 9175 |
| Sample 7 bladder cancer | 2531 | 3127 | 7511 | 9480 | 12321 |
| Sample 8 bladder cancer | 3009 | 3047 | 5670 | 6240 | 9686 |

(continued)

| Diagnosis | CA, U/mL - 0 min | CA, U/mL - 3 min | CA, U/mL - 10 min | CA, U/mL - 20 min | CA, U/mL - 30 min |
|---|---|---|---|---|---|
| Healthy control 1 | 2835 | 3545 | 3277 | 3312 | 3294 |
| Healthy control 2 | 2664 | 2617 | 2628 | 2658 | 2638 |
| Healthy control 3 | 2807 | 2836 | 2816 | 2872 | 2877 |
| Healthy control 4 | 2965 | 2977 | 2956 | 2978 | 2987 |
| Healthy control 5 | 2578 | 2860 | 2871 | 2881 | 2898 |
| Healthy control 6 | 3261 | 3334 | 3287 | 3278 | 3301 |
| Healthy control 7 | 3556 | 3583 | 3528 | 3567 | 3579 |
| Healthy control 8 | 3170 | 3189 | 3128 | 3177 | 3192 |

Conclusion: The results obtained in this example prove that new cancer antigen is generated by the way the serum samples are processed, since without the use of heat treatment and an acidic reaction medium, there is no difference between healthy controls and samples from cancer patients.

Example 2

In addition to obtaining a cancer antigen in the serum of patients with carcinomas, it can also be obtained in saliva by the method described in this invention, as shown Figure 6. Although the accuracy of the saliva immunoassay is lower than that of serum, saliva collection has the advantages of being a simple and inexpensive way to collect biosamples.

Frozen saliva samples of patients in 1.5 ml Eppendorf tubes were thawed and brought to room temperature, after which they were centrifuged in a microcentrifuge at 16000 rpm. Then the samples were subjected to heat treatment in a water bath in the temperature range from 50 to 65 °C for 30 minutes. The immunoassay for saliva, based on competitive binding, included the main parameters: a) the use of less than the amount of luminescent cancer antigen conjugate 55-85 kDa (~ 30-50 ng / ml versus 100-200 ng / ml); b) high concentration of saliva samples {1/2; 1/5; 1/10; and 1/20}. The saliva samples were divided into 2 groups for the study: 1 group was heated for 30 minutes in a water bath in the temperature range from 50 to 65 ° C (the same as the serum samples), and the other group was left unheated.

To perform immunoassay of saliva samples, 9 $\mu$g / ml of monoclonal antibodies MAT IgMEC in PBS buffer pH 7.5 were sorbed onto the plates. The plates were incubated overnight at 4 °C and then washed 3 times with distilled $H_2O$. Then, the non-specific binding of plates with adsorbed antibodies was blocked with 200 $\mu$l per well of dicarboxylic acid blocking buffer solution, pH 4.0, incubated for 2 hours at 37 °C, washed 3 times with distilled $H_2O$, frozen and freeze-dried. To the wells of a lyophilized plate Ns 1, 25 $\mu$l aliquots of heat-treated saliva samples from patients with breast cancer and from healthy controls and 25 $\mu$l of blocking buffer solution pH 4.0 were added. In a lyophilized Ne 2 plate, 25 $\mu$l of unheated saliva samples and saliva from healthy controls mixed with 25 $\mu$l of blocking buffer were added to the wells. Finally, 50 $\mu$l of Series N ° 41 cancer antigen (PA-Acridine # 41) at a final concentration of 30 ng/ml was added to all wells of both plates. The reaction solutions were thoroughly mixed by tapping on all sides of the plate, and then incubated at room temperature for 2 hours. After incubation, all solutions were aspirated, washed three times with distilled $H_2O$, the results were read with a flash chemiluminometer.

Results obtained: Good discrimination was obtained for saliva samples that underwent special heat treatment (AUC = 0.894), comparable to those for serum. The ratio between patients and healthy controls was 4.6. Using a smaller amount of PA-Acridine conjugate allowed distinguish saliva samples better than the usual concentration range of 100-200 ngml used for serum.

Conclusion: The saliva test can serve as the primary patient selection method using a threshold low enough to detect the majority of patients with suspected cancer. Primary patients thus selected with an elevated level of cancer antigen in their saliva will be referred for a serum test with a higher threshold t to rule out false positives.

Example 3. A method of isolating a cancer antigen from an extract of myeloid leukemia cancer cells.

The human chronic myelogenous leukemia cell line K-562, obtained from the American collection of cell cultures ATTC, N ° CCL-243, was placed in 100 ml of medium containing 5x105 cells/ml in an 850 ml roller bottle. The cancer cells were then suspended in a suitable cell culture medium, for example, Dulbecco's as modified by Iskov. The bottles were kept at 37 °C without $CO_2$ with rotation in the range 0.25-1.0 rpm. The cells were decanted, washed and resuspended in 50 mM Tris buffered saline (TBS) buffer pH 7.4 to obtain a suspension containing $50 \times 10^6$ cells/ml. The resulting tubes with cells were frozen at -20 °C until the cell extract was prepared.

Then, a cell extract was prepared. Briefly, cells were subjected to ultrasonic sonification on ice. The resulting extract was centrifuged in Eppendorf tubes at 16,000 g in a rotor with a fixed angle for 10 min. The resulting supernatant is a K-562 extract, which was collected into a clean test tube. The total protein concentration in the cell extract was determined according to the standard Bradford method using Comassie G250 and BSA as a standard. The protein concentration was in the range of 5-10 mg/ml. Then, the isolation of cancer antigen was carried out on a prepared Sepharose column-4B, C-9142 (Sigma-Aldrich) with immobilized native porcine AFP by affinity chromatography. D cyan bromide Sepharose was used as a carrier. After removing the buffer solution, 30-40 ml of K-562 supernatant was added to the column and left in the column overnight at + 40 ° C. Mixing the supernatant with immobilized sorbent was carried out on a special rocking shaker with constant oblique stirring.

The next day, the column was washed with TBS buffer until a stable baseline signal was obtained at A280. On a peristaltic pump of a low pressure liquid preparative chromatograph, the rate was set to 1.5 = 2.0 ml/min, preferably 1.8 ml/min. The passage of the extract through the column was monitored using optical UV detection at 280 nm.

Elution of the cancer antigen was carried out using a Tris-HCl with KCl at a concentration of 0.5 to 1.5 mol/L, preferably 1 mol/L. Antigen was collected when the optical instrument recorded a shift from the baseline signal until it peaked and until the signal returned to baseline. Then the protein collection was stopped. Thereafter, the column was equilibrated by washing with a 5-fold column volume with TBS-buffered solution. Then, the extract that did not initially adhere to the column was again passed through the column with the immobilized ligand and stirred in the column with the sorbent at + 40 °C overnight, after which the procedure for elution of the cancer antigen described above was repeated. This procedure was repeated 3-4 times until the complete removal of the cancer antigen from the extract.

All protein eluates obtained from one extract were combined into one preparative fraction, which was dialyzed against 1x PBS. The obtained purified protein was transferred into a weighed 50 ml sterile plastic Falcon tube, frozen at -20 ° C for 12 hours until the sample was deeply frozen, after which the frozen sample was freeze-dried for 36 hours. Then the protein concentration in the lyophilisate was determined by the Bradford method and the purity of the isolated antigen by polyacrylamide gel electrophoresis (Figure 7)

Figure 7. Polyacrylamide gel electrophoresis and Western blotting show a glycoprotein with a molecular weight of 55-85 kU), isolated in pure form from a suspension of human chronic myelogenous leukemia K-562 cancer cells, is recognized by specific monoclonal antibodies IgMEC against cancer antigen.

Conclusion: The described purification method using immunoaffinity chromatography with native porcine AFP as a ligand allows the isolation of a pure cancer antigen, which is recognized by monoclonal antibodies against the specified cancer antigen Example 4.

Confirmation of the presence of an identical marker protein, N-glycoprotein with a molecular weight of 55-85 kDa, in homogenates of various malignant tumors (carcinomas) was carried out by Western blotting using mAb (monoclonal antibodies) against the N-glycoprotein. The presence of carcinoma-specific antigen, a marker protein for epithelial carcinomas, has been identified in various human epithelial tumors are shown in Table 2:

Table 2. Quantification of carcinoma-specific marker in homogenates of various epithelial tumors

| # | Type of epithelial tumor cell line | Antigen amount, carcinoma-specific marker, Unit/mL* |
|---|---|---|
| 1 | Breast cancer carcinoma MCF-7 and T47D | 9500-12787 |
| 2 | Hepatocellular carcinoma HEPG2 | 4800 |
| 3 | Ovarian carcinoma SCOV3 | 11780 |
| 4 | Stomach carcinoma NCI-N87 | 12152 |
| 5 | Lung carcinoma NCI-H1385 | 10192 |
| 6 | Colorectal adenocarcinomas LoVo and HCT-116 | 8720 -11210 |
| 7 | Prostate cancer adenocarcinomas 22Rv1 and LNCaP | 9879 -11222 |
| 8 | HeLa cell line from cervix adenocarcinoma | 22317 |
| 9 | Rectum adenocarcinoma SW-620 | 4880 |

(continued)

| # | Type of epithelial tumor cell line | Antigen amount, carcinoma-specific marker, Unit/mL* |
|---|---|---|
| 10 | K-562 cell line | 11277 |

* Data published in: Janneta Tcherkassova, Sergei Tsurkan, Galina Smirnova, Julia Borisova, Ricardo Moro and Helen Treshalina. Binding characterization of the targeting drug AIMPILA to AFP receptors in human tumor xenographts. Tumor Biology 2017: online access P. 1-11

A carcinoma-specific antigen with a molecular weight of 55-85 kDa was isolated from the MCF human breast adenocarcinoma tumor extract using affinity chromatography. Immunization of Balb / c mice with an isolated glycoprotein led to the development of acute phase Mab-IgM monoclonal antibodies that recognize the MEC polysaccharide fragment. Based on the isolated antigen and the Mab-IgM monoclonal antibodies, immunochemical serum and suspension tests were subsequently developed to quantify a marker for epithelial carcinomas. For the first time, a quantitative determination of the level of the marker of epithelial carcinomas MEK was carried out on models of transplanted human tumors growing in s/c xenografts using a suspension ICA test on monoclonal antibodies.

Preparation of human tumor homogenates: Fragments of a subcutaneous tumor of the tumor node were excised with a scalpel, transferred to a cryotube, and after adding 1 ml of TBS buffer, ultrasonic sonification of the tumor mass was performed using an ultrasonic homogenizer from BILON-150Y (China) at a set power of 60 W (40%). Sonication was carried out on ice for 6 times , each 30 sec long with two 30 sec stops until a homogeneous mixture was obtained, after which the material was centrifuged at 13000 rpm in an Eppendorf centrifuge for 10 min at room temperature. The resulting supernatant was a tumor tissue homogenate ready for analysis. The total protein concentration in human tumor samples MCF-7, T47D, HEPG2, SCOV3, NCI-N87, LoVo and HCT-116, 22Rvl and LNCaP, HeLa, SW-620 and K-562 was determined according to the standard Bradford method. Western blot of homogenates of the studied tumors showed the presence of a single cancer antigen, specifically recognized by monoclonal antibodies.

Conclusion: The specificity of carcinoma-specific antigen was tested using various human epithelial tumors obtained from s.c. xenografts in nude Balb/c mice. All studied human epithelial tumors of various localizations (carcinomas) contain an identical N-glycoprotein with a molecular weight of 55-85 kDa, which is recognized by specific monoclonal antibodies against this cancer antigen. The glycoprotein is specific only to epithelial carcinomas.

Example 5:

Confirmation that the new marker is a glycoprotein was obtained in experiments with periodate oxidation of polysaccharides, which was carried out at room temperature with 0.1 N sodium periodate solution for 2 hours. With periodate oxidation, 1.3 mol of sodium periodate is consumed per 1 mol of polysaccharide, while 0.5 mol of formic acid is released. After reduction with sodium borohydride (NaB4), binding to alpha-fetoprotein was performed to confirm hypotheses about the presence of a glycoside, which is an active epitope of glycoprotein binding.

Our experiments with sodium periodate showed that alpha-fetoprotein binds to the N-glycosidic fragment associated with the protein part of the glycoprotein molecule. The wells of the microtiter plate were coated with a homogenate of cancer cells of chronic myeloid leukemia K562 or human serum albumin (HSA), after which the absorbed proteins were treated with various concentrations of sodium periodate (from 1 to 100 mmol / L). Subsequent incubation with biotinylated AFP demonstrated the effect of periodate oxidation on AFP binding. The binding activity of the studied glycoprotein was tested using 1 $\mu$g/ml AFP-Biotin. As shown in Figure 10, treatment of the cancer cell homogenate with sodium periodate results in a significant decrease in active binding (up to 90%), this decrease being proportional to the periodate concentration.

Human serum albumin HSA, which does not contain polysaccharides, was used as a control. The control wells of the plate, with HSAwas absorbed on the plate at the same concentration as K-562 and treated with sodium periodate. The control experiment showed no decrease in the binding of albumin with monoclonal antibodies against HSA, regardless of the concentration of sodium periodate used. The integrity of the albumin control protein was verified using monoclonal antibodies against human albumin. AFP-Biotin and anti-HSA mAb were detected by binding to streptavidin-horseradish peroxidase and anti-IgG-HRP, respectively.

Conclusions: As shown in Figure 10, AFP-Biotin binding decreases with increasing periodate concentration, reaching a plateau above 10 mmol/L. At the same time, the absence of the effect of periodate on HSA is obvious, which indicates that sodium periodate has an effect not on the peptide chain of a glycoprotein, but on its hydrocarbon moiety (glycosides).

5.1. Purified antibodies against cancer antigen and AFP lose their ability to bind to the active antigen epitope in the MCF-7 cell culture homogenate after treatment with sodium periodate.

The MCF-7 breast adenocarcinoma cancer cells homogenate at a concentration of 100 $\mu$g / ml was absorbed onto the wells of a microtiter plate.. The wells were washed with PBS with 0.02% Tween-20 and 11 two-fold dilutions of sodium periodate were added to the wells, starting at 100 mmol / L in 0.250 mol / L Malonate buffer, pH 4.0. After 2 hours incubation at room temperature (in the dark), the sodium periodate solution was aspirated, after which 150 $\mu$l of 2% glycine in PBS was added to the wells of the plate to block aldehydes and the solution was kept in the wells for 2 hours at room temperature. Then the wells of the plate were blocked with 250 $\mu$l of 1% BSA in PBS overnight at 4 °C. The wells were washed 3 times, after which monoclonal antibodies IgMEC, polyclonal rabbit antibodies against the specified cancer antigen or AFP-Biotin were incubated in the wells for 1 hour at room temperature. After washing, the corresponding secondary antibody or streptavidin-peroxidase conjugate was added to the wells of the plate and incubated for another hour. Then the wells were washed, ABTS substrate was added (Diammonium salt of 2,2'-Azino-bis- (3-ethylbenzothiazoline-6-sulfonic acid), which is a water-soluble substrate of horseradish peroxidase that produces a green color of the final reaction product. Then the plate was read at 405 nm on a standard ELISA plate reader The experimental results are shown in Figure 11.

Conclusions: Monoclonal antibodies IgMEC against the cancer antigen, as well as AFP, do not recognize the glycoprotein on the MCF-7 homogenate, which was oxidized with sodium periodate. Sodium periodate did not change all the binding sites on the polyclonal antibodies, which were still able to recognize the binding sites on the MCF-7 homogenate. In contrast to polyclonal antibodies, an increase in the concentration of sodium periodate resulted in a decrease in the binding of AFP and IgMEC to the homogenate of MCF-7 cells. This can only be explained by the destruction of polysaccharides as a result of periodate oxidation, which confirms that the cancer antigen binding epitope is a glycoside.

5.2 Periodic oxidation of glycosides in patient serum samples:

For this experiment, 32 serum samples from 16 healthy and 16 verified prostate cancer patients were used. To each serum sample of 50 $\mu$l volume, 5 $\mu$l of a 1 mol/L solution (100 mmol/L) sodium periodate NaIO$_4$ (Molar mass: 213.8918 g / mol) was added. The mixture was incubated for 60 minutes at room temperature. Thereafter, 10 $\mu$l of 0.5 mol/L polyethylene glycol (PEG) solution was added to each well to remove excess periodate. Immunochemiluminescence analysis by the principle of competitive binding was carried out with IgMEC adsorbed on Mab plates against cancer antigen, glycoprotein 55 - 85 kDa using 100 ng/ml luminescent conjugate of cancer antigen-62-Acridine. Incubation time: 2 hours at room temperature. The results are presented in Table 3.

TABLE 3. The results of the sodium periodate oxidation of glycosides in serum samples

| Samples | Diagnosis | Sodium periodate treatment | Result | Sodium periodate treatment | Result |
|---------|-----------|---------------------------|--------|---------------------------|--------|
| N121 | Healthy control | Yes | 178 | No | 1018 |
| N122 | Healthy control | Yes | 182 | No | 948 |
| N123 | Healthy control | Yes | 218 | No | 1014 |
| N124 | Healthy control | Yes | 120 | No | 808 |
| N125 | Healthy control | Yes | 222 | No | 950 |
| N126 | Healthy control | Yes | 174 | No | 1116 |
| N127 | Healthy control | Yes | 180 | No | 1064 |
| N128 | Healthy control | Yes | 188 | No | 898 |
| N129 | Healthy control | Yes | 142 | No | 838 |
| N130 | Healthy control | Yes | 172 | No | 842 |
| N131 | Healthy control | Yes | 158 | No | 980 |
| N132 | Healthy control | Yes | 226 | No | 866 |
| N133 | Healthy control | Yes | 78 | No | 788 |
| N134 | Healthy control | Yes | 118 | No | 896 |
| N135 | Healthy control | Yes | 160 | No | 1040 |
| N136 | Healthy control | Yes | 178 | No | 954 |

(continued)

| Samples | Diagnosis | Sodium periodate treatment | Result | Sodium periodate treatment | Result |
|---------|-----------|---------------------------|--------|---------------------------|--------|
| P-3031 | Prostate cancer | Yes | 112 | No | 480 |
| P-3032 | Prostate cancer | Yes | 276 | No | 400 |
| P-3033 | Prostate cancer | Yes | 298 | No | 648 |
| P-3034 | Prostate cancer | Yes | 196 | No | 436 |
| P-3035 | Prostate cancer | Yes | 186 | No | 572 |
| P-3036 | Prostate cancer | Yes | 268 | No | 384 |
| P-3037 | Prostate cancer | Yes | 124 | No | 444 |
| P-3038 | Prostate cancer | Yes | 118 | No | 472 |
| P-3039 | Prostate cancer | Yes | 150 | No | 508 |
| P-3040 | Prostate cancer | Yes | 184 | No | 560 |
| P-3041 | Prostate cancer | Yes | 140 | No | 468 |
| P-3042 | Prostate cancer | Yes | 116 | No | 592 |
| P-3043 | Prostate cancer | Yes | 136 | No | 416 |
| P-3044 | Prostate cancer | Yes | 224 | No | 440 |
| P-3045 | Prostate cancer | Yes | 210 | No | 460 |
| P-3046 | Prostate cancer | Yes | 244 | No | 524 |

The results of the experiment showed that samples subject to periodate oxidation, which destroys the activity of glycosides, showed no difference between patients and healthy, in contrast to control samples, which were not treated with sodium periodate (Figures 12 and 13).

Conclusion: Periodic oxidation of polysaccharides, which are an integral part of the marker protein, leads to a loss of cancer antigen activity. The above examples confirmed the hypothesis that the active determinant of the cancer antigen, the glycoprotein with MW 55-85 kDa, is precisely the carbohydrate fragment of the glycoprotein covalently linked to the protein through the glycosidic moiety.

5.3 Enzymatic hydrolysis of peptide bonds in protein molecules.

To further confirm that only the polysaccharide fragment is involved in binding, and not the peptide chain of the glycoprotein, the following experiment was carried out: the biomaterial containing the glycoprotein was subjected to complete enzymatic cleavage by pepsin, after which its activity was assessed by inhibition of binding. Pepsin hydrolyzes peptide bonds formed by aromatic amino acids - tyrosine and phenylalanine with the highest rate, however, unlike other proteolytic enzymes, trypsin and chymotrypsin, it does not have a strict specificity. Digestion was carried out for 24 hours in a solution using 0.04% pepsin at 37 °C, pH 2.5 (the control PAGE gel electrophoresis of the digested biomaterial did not show any bands, except for the band of pepsin itself). Since the cleavage of the glycoprotein adsorbed in the wells of the plate leads to the cleavage of peptide fragments and therefore they would not bind to the plastic, the assay was performed as follows: After raising the pH, a 1 μg / ml AFP-Biotin solution was mixed with various concentrations of the pepsin-digested MCF-7 extract. This mixture was then added to wells coated with the same extract, but without pepsin digestion. Thus, if the binding epitope is a peptide fragment of a glycoprotein, the concentration of the attached AFP-Biotin will be directly proportional to the amount of uncleaved glycoprotein. As a control, the same glycoprotein preparation was used, processed in the same way as described above, but without pepsin. Another control experiment was performed using human albumin and anti-albumin monoclonal antibodies.

Figure 14 shows that the inhibition curve obtained with the pepsin-cleaved homogenate of MCF-7 cancer cells was substantially identical to that of the non-cleaved homogenate. Pepsin cleavage of MCF-7 homogenate did not result in a difference in binding to AFP-Biotin. This does not apply to controls with human serum albumin, which showed a large difference in optical values of density between cleaved pepsin and non-cleaved protein.

Thus, the experiment with enzymatic cleavage of proteins unambiguously confirmed that the active epitope of glycoprotein binding is the polysaccharide fragment itself, and not the protein part of the molecule.

Conclusion: The experiment carried out confirmed that the analyzed glycoprotein with M.W. 55-85 kDa in the composition of the homogenate of cancer cells MCF-7 contains a polysaccharide fragment, which is responsible for specific binding to Alpha-fetoprotein and does not depend on the composition of the protein.

5.4 Enzymatic digestion of serum samples from healthy controls and cancer patients.

Serum samples (8 healthy controls and 8 serum samples from cancer patients) were centrifuged at 16,000 rpm for 10 minutes. To 100 μL of the sample, 100 μL of 500 mmol L Glycine-HC1, pH 3.0, containing 0.08% pepsin was added. Using pH indicator paper, it was verified that in the presence of the enzyme, the serum had a pH of 4.0 or lower. Then the samples were placed in an incubator at 37 °C for 18 hours. Enzymatic hydrolysis was stopped by the addition of 1 mol/L Tris buffer pH 7.5, which was confirmed by the presence of neutral pH with an increase in the initial sample volume (100 μL) to 250 μL. Serum samples subjected to enzymatic hydrolysis by pepsin were run on 10% r polyacrylamide gel electrophoresis of proteins in the presence of sodium dodecyl sulfate according to Laemmli's method (SDS PAGE) under non-denaturing conditions. For this, before being loaded onto the gel, samples in a volume of 20 μl / well were boiled in the presence of sodium dodecyl sulfate (SDS) and 2-mercaptoethanol. To visualize the results of electrophoresis, proteins were stained in the gel with Coomassie blue.

SDS polyacrylamide gel electrophoresis shows 6 serum samples before and after pepsin digestion (3 BC samples and 3 healthy controls). Obviously, most of the protein has disappeared, with the exception of the pepsin band.

If the glycoprotein binding site is a glycoside rather than a peptide, we would expect to see the same difference between patients and healthy controls as before pepsin digestion (the expected values should somehow differ due to the dilution factor of the samples). Table 4 below shows the numerical values of RLU (Relative Light Units) obtained with the same samples before and after their treatment with pepsin.

Table 4. Relative light units for the pepsin- treated and untreated samples.

| Sample # | Diagnosis | RLU | |
| --- | --- | --- | --- |
| | | No Pepsin | + Pepsin |
| 1430 | Healthy control | 5040 | 4230 |
| 955 | Healthy control | 4520 | 4840 |
| 1335 | Healthy control | 3540 | 3440 |
| 1424 | Healthy control | 3519 | 3490 |
| 1410 | Healthy control | 3220 | 4490 |
| 1300 | Healthy control | 3420 | 4180 |
| 31-03 | Breast cancer | 1460 | 1640 |
| 164-04 | Breast cancer | 2200 | 1500 |
| 19-03 | Breast cancer | 2220 | 1610 |
| 63-03 | Breast cancer | 1380 | 1830 |
| 293-04 | Breast cancer | 2080 | 1980 |
| 281-04 | Breast cancer | 1420 | 2170 |

Table 5 The statistic data of immunoassay of samples treated by pepsin in compare with untreated samples.

|  | No Pepsin | + Pepsin |
|---|---|---|
| Healthy controls | 3877 | 4112 |
|  |  |  |
| Breast cancer | 1793 | 1788 |
|  |  |  |
| Healthy/ Cancer ratio | 2.2 | 2.2 |

Conclusions:

1. From Table 5 and Figure 16 it follows that the ratio of healthy controls to breast cancer patients, as well as the difference between samples, remains unchanged with or without pepsin digestion. This confirms our hypothesis that the active epitope of the cancer antigen is not the protein part of the molecule, but its N-glycosidic fragment.

2. Generalized analysis of the results of studies on the periodic oxidation of glycosides and enzymatic degradation of proteins by pepsin with the use of serum samples and other biological materials indicates that: a) The results obtained by immuno-chemiluminescence analysis using MAT IgMEC are consistent with the observations made regarding the binding of the specified cancer antigen to AFP, as well as to IgMEC. b) The immunoassay is based on the glycosidic portion of the cancer antigen and NOT on the peptide chain.

The sequence of the protein in this case is completely irrelevant, since regardless of which protein or proteins contain it, the glycosidic fragment is quantitatively measured, which is the active antigenic determinant of the specified cancer antigen. The current state of the art does not allow determining the structure and sequence of the branched structure of the N-glycoside that binds to the antibody.

3. The glycosidic nature of a glycoprotein with an affinity for AFP and a molecular weight of 55-85 kDa was established using various methods: periodate oxidation of polysaccharides, which destroy their activity, and enzymatic hydrolysis of peptide bonds in protein molecules using the proteolytic enzyme pepsin with the formation of more simple peptides and free amino acids.

Example 6 Inhibition of N-glycosylation using tunicamycin.

To confirm the N-glycosylation of cancer antigen, a glycoprotein from M.W. 60-65 kDa in cancer cells, cancer cell homogenates were incubated with tunicamycin, which specifically blocks N-linked glycosylation. Homogenates of MCF-7 breast adenocarcinoma cancer cells previously incubated with tunicamycin were dispensed into the wells of an ELISA plate, followed by incubation with 10 μg/ml AFP-HRP for 2 hours at room temperature. The binding activity of AFP to cancer cells treated with tunicamycin was significantly lower compared to untreated control cells as shown in Figure 17.

From Figure 17 it is evident that treatment of the specified glycoprotein with tunicamycin resulted in a 90% decrease in the binding of AFP to the MCF-7 homogenate. At the same time, the treatment of cells with galactosamine, which inhibits the binding of O-glycosides, did not lead to a change in binding. Conclusion: These results unambiguously confirm that the analyzed glycoprotein with M.W. 55-85 kDa in the composition of the homogenate of cancer cells MCF-7 contains precisely N-glycoside, which is the AFP binding epitope.

Example 7:

To determine the diagnostic sensitivity and specificity of the test using the isolated cancer antigen specific for epithelial carcinomas, 300 verified serum samples were analyzed in blinded clinical and laboratory studies, including 58 blood serum samples from patients with colorectal cancer, 25 serum samples from patients with breast cancer, 32 serum samples from lung cancer patients, 35 serum samples from prostate cancer patients, and 150 verified serum samples from healthy individuals used as controls of appropriate age and sex. The results of the studies performed are presented in Figure 18.

Determination of the diagnostic sensitivity

Diagnostic (clinical) sensitivity was defined as the percentage of truly positive results, confirmed by histology. To calculate the "true" value of clinical (diagnostic) sensitivity, a formula was used in accordance with GOST R 53022.3-2008 (Table 4, Section 5.5) and GOST R ISO 18113-1-2015 Section A.3.15):

$$Sens\% = TP/(TP+FN),$$

where

TP - true positive test result confirmed by histopathology,
FN - false negative test result confirmed by histipathology.

Thus, the "true" value of the diagnostic sensitivity at 95% specificity was 93.7% (see Table 2).
To calculate the interval in which the "true" value of the diagnostic sensitivity and specificity is located with a confidence level of C = 95%, that is, to determine the indicator of the correctness of the studies, the formula was used in accordance with GOST R 53022.3-2008 and the probability theory for independent tests by Bernoulli distribution*:

$$D\% = D0\% \pm 1.96 * [D0\% *(100- D0\%)/n](1/2),$$

where

Do% is the target value of the relative displacement value;

D% is the target value of the coefficient of total analytical variation;

1,96 is the quantile of the standard normal distribution for a significance level of 0.05.

* Bernoulli's distribution in probability theory and mathematical statistics is a discrete probability distribution that simulates a random experiment of an arbitrary nature, with a known probability of success or failure. B.I. Polozhintsev. Theory of Probability and Mathematical Statistics. Introduction to Mathematical Statistics: A Study Guide. - SPb.: Publishing house of Polytechnic University, 2010.-- 95 p.

The interval in which the "true" value of the diagnostic sensitivity is located with a confidence level of C = 95% was 89.8-97.6%.

Determination of the diagnostic specificity

The diagnostic specificity of the test was determined as the proportion, expressed as a percentage, of truly negative serum samples from healthy people with a confirmed absence of cancer. To calculate the "true" value of clinical (diagnostic) specificity, a formula was used in accordance with GOST R 53022.3 - 2008 (Table 4, Section 5.5) and GOST R ISO 18113-1-2015 (Section A.3.16):

$$Sp\% = TN / (TN + FP),$$

where

TN - true negative test result,

FP - false positive test result.

Thus, the "true" value of the diagnostic specificity was 98.7% (see Table 7). The interval in which the "true" value of diagnostic specificity is located with a confidence level of C = 95% was 96.9-100.0%.

Table 7. Results of studies of 300 clinical serum samples from healthy controls and cancer patients using cancer antigen 55-85 kDa.

| Criterion | Carcinomas of various locations | Healthy controls |
|---|---|---|
| Total amount of samples N = 300 | N = 150 | N = 150 |
| Positive result | Truth positive (TP) N = 140 | False positive (FP) N = 2 |
| Negative result | False negative (FN) N= 10 | Truth negative (TN) N = 148 |
| Diagnostic Sensitivity | 93.7% Proportion of truth positive results in Groupe | Range of 95% confidence interval |
| Diagnostic Specificity | Proportion of truth negative results in Groupe | 98.7% |
| Positive predicted value (PPV) Proportion of truth positive results from all positive results | 98.5% (TP/TP+FP)*100% | |
| Negative predicted value NPV | 93.7% (TN/TN+FN)*100% | |
| Test diagnostic efficacy % Proportion of truth positive results from all results | 96% (TP+TN)/(TP+FN+TN+FP)*100% | |

Conclusions: Previous studies on various groups of serum samples, cancer patients, using the sample processing method described in this invention, made it possible to separate patient samples from healthy controls and patients with various benign diseases (data not shown) from cancer patients with high sensitivity and specificity. At the same time, for serum samples of patients with cancer, the highest concentration of the specified cancer antigen was observed at the earliest stages of the development of the disease, which distinguishes the new cancer antigen from other tumor markers. It is this feature of the specified cancer antigen that can be used for screening studies among a conventionally healthy population.

BIBLIOGRAPHY:

1. Severin SE, Moskaleva EY, Shmyrev II, et al. Alpha-fetoprotein-mediated targeting of anticancer drugs to tumor cells in vitro. Biochem Mol Biol Int 1995; 37: 385-392.

2. Cancer V. Compositions of Alpha-fetoprotein and inducers of apoptosis for the treatment of cancer. Google Patents, 2008, http://www.google.com.na/patents/US20080318840).

3. Janneta Tcherkassova, Sergei Tsurkan, Galina Smirnova, Julia Borisova, Ricardo Moro and Helen Treshalina. Binding characterization of the targeting drug AIMPILA to AFP receptors in human tumor xenographts. Tumor Biology 2017: online access P. 1-11).

4. Sergei Tsurkan, Janneta Tcherkassova, Vera Gorbunova, Helen Treshalina, Elena Yu. Grigorieva. New drug AIMPILA targeted to AFP receptor: Oral anticancer therapy and biodistribution in vivo. Journal of Clinical Oncology. 2018. Volume 36, Issue 15_suppl, p. e24232

5. D. M. Weir & CC. Blackwell, eds. Handbook of Experimental Immunology 1987; 4th edition,., Blackwell Science Inc.,

6. F. M. Ausubel et al., Eds. Current Protocols in Molecular Biology 1987.

7. Spiro, A., Lowe, M. and Brown, D. A Bead-Based Method for Multiplexed Identification and Quantitation of DNA Sequences Using Flow Cytometry. Appl. Env. Micro. 66, 4258-4265 (2000).

8. Crowther, J. R. The ELISA guidebook. Humana Press: New Jersey (2000);

9. Harlow, E. and Lane, D., Using antibodies: a laboratory manual. Cold Spring Harbor Laboratory Press: Cold Spring Harbor (1999).

**Claims**

1. A method of detection of the presence and/or concentration (expression level) of a cancer antigen (a marker for epithelial carcinomas), including: (a) obtaining blood samples, tumor tissue, cancer cell culture, bone marrow tissue, saliva from patients with suspected malignant epithelial neoplasms; (b) obtaining samples of blood, saliva from a control group of healthy people without malignant neoplasms; (c) a method for obtaining a new marker for epithelial carcinomas formed on the surface of cancer cells of epithelial genesis; (d) a method for isolating a marker for epithelial carcinomas; (e) if the expression level of the marker for epithelial carcinomas in the indicated samples in (a) exceeds the expression level of the a marker for epithelial carcinomas in (b), then the result means a high probability of the epithelial cancer presence.

2. The method as defined in Claim 1, is used for obtaining a new marker for epithelial carcinomas by: (a) obtaining samples of serum, blood, tumor tissue, culture of cancer cells, bone marrow tissue, saliva of patients with suspected malignant epithelial neoplasms; (b) using thermal treatment of the biological fluids samples in the range of 50 to 65 °C from patients or cancer cell lines in culture; (c) using an acidic environment with a low pH in the 2.0 to 5.0 range.

3. The method as defined in Claim 1, used for the isolation of a cancer antigen, the marker for epithelial carcinomas) consisting of: (a) obtaining a suspension of cancer cells; (b) processing the cell suspension and making a cell extract; (c) isolation and purification of the cancer antigen from the cell extract by immunoaffinity chromatography using porcine or other suitable species of alpha-fetoprotein.

4. The cancer antigen obtained according to the methods as described in Claims 2 and 3 comprising a family of N-glycoproteins having identical N-glycosylation, with a molecular weight between 55 and 85 kDa.

5. The method as defined in Claim 1, wherein the specified determination of the concentration measurement of the marker for epithelial carcinomas is carried out using any type of immunoassay method (direct or competitive binding, sandwich assay, and others).

6. The method as defined in Claim 1 is then used for determination of the normal range of values (norm) of the expression level of the marker for epithelial carcinomas (or concentration measurement) in a control group of healthy people without malignant neoplasms.

7. The method as defined in Claim 1, wherein the indicated determination of the expression level, or concentration measurement of the cancer antigen, is performed using monoclonal or polyclonal antibodies of animal origin directed against the N-glycosidic part of the N-glycoprotein family specified in Claim 6.

8. The method as defined in Claims 1 and 5, wherein the specified antibody can be a polyclonal antiserum against the specified cancer antigen, the marker for epithelial carcinomas.

9. The method as defined in Claims 1 and 5, wherein said determination of the expression level (or concentration) of the cancer antigen is performed using antibody-derived oligopeptides.

10. The method as defined in Claim 5, wherein the described test sample is obtained from the blood.

11. The method as defined in Claim 5, wherein the described test sample is obtained from cancer cell lines, a suspension of tumor-derived tissue, bone marrow tissue, and saliva.

**12.** A method for detecting epithelial cancers using a cancer antigen according to Claim 1, comprising of:

a) a capture reagent of said cancer antigen, which may be an oligopeptide or an antibody specific for a particular sequence of N-polysaccharides of said cancer antigen;

b) a detection reagent capable of detecting a cancer antigen associated with said capture reagent upon interaction with said cancer antigen;

c) a detection reagent capable of detecting said cancer antigen associated with said capture reagent such as a luminescent, bioluminescent, enzymatic, fluorescent, or radioactive label attached to the isolated antigen.

Figure 1

| n | 16 |

| Diagnosis | n |
|---|---|
| Normal control | 8 |
| Bladder Cancer | 8 |

| Curve | Area | SE | p | 95% CI of Area | Diagnosis = Bladder Canc |
|---|---|---|---|---|---|
| RECAF u 3 min | 0,531 | 0,1569 | 0,4211 | 0,224 to 0,839 | have lower values |
| RECAF u 20 min | 0,656 | 0,1611 | 0,1661 | 0,340 to 0,972 | have higher values |
| RECAF u 30 min | 0,984 | 0,0248 | <0.0001 | 0,936 to 1,000 | have higher values |

| Contrast | Difference | p |
|---|---|---|
| CAF u 3 min v RECAF u 20 min | -0,125 | - |
| CAF u 3 min v RECAF u 30 min | -0,453 | 0,0031 |
| AF u 20 min v RECAF u 30 min | -0,328 | 0,0425 |

Figure 2

Figure 3

| n | 40 |
|---|---|

| Diagnosis | n |
|---|---|
| Normal control | 20 |
| Colorectal cancer | 20 |

| Curve | Area | SE | p | 95% CI of Area | Diagnosis = Colored |
|---|---|---|---|---|---|
| 1 in 10 RECAF 50 ng | 0,716 | 0,0817 | 0,0040 | 0,556 to 0,876 | have higher value |
| 1 in 10 RECAF 100 ng | 0,693 | 0,0832 | 0,0104 | 0,529 to 0,856 | have higher value |

| Contrast | Difference | p |
|---|---|---|
| F 50 ng v 1 in 10 RECAF 100 ng | 0,024 | 0,7504 |

Figure 4

Figure 5

Figure 6

Figure 7

Figure 8

Figure 9

Figure 10

Figure 11

Figure 12

The effect of periodate oxidation on the difference between cancer patients and healthy controls

Figure 13

Figure 14

1 - empty well
2-7 - cancer samples; 8-13 - healthy controls;
3, 5, 7, 9, 11, 13 - samples after pepsin treatment;

Figure 15

Figure 16

Figure 17

Figure 18

Figure 19

Figure 20

## INTERNATIONAL SEARCH REPORT

| International application No. |
| --- |
| PCT/RU 2020/000250 |

### A. CLASSIFICATION OF SUBJECT MATTER
G01N 33/532 (2006.01); G01N 33/574 (2006.01); C12N 5/09 (2010.01)

According to International Patent Classification (IPC) or to both national classification and IPC

### B. FIELDS SEARCHED

Minimum documentation searched (classification system followed by classification symbols)
G01N 33/50, C12N 5/09, C12Q 1/6886, G01N 33/532, G01N 33/574

Documentation searched other than minimum documentation to the extent that such documents are included in the fields searched

Electronic data base consulted during the international search (name of data base and, where practicable, search terms used)
EAPATIS, ESPACENET, PatSearch (RUPTO internal), Information Retrieval System of FIPS, USPTO, PATENTSCOPE, E-Library, NCBI, Google

### C. DOCUMENTS CONSIDERED TO BE RELEVANT

| Category* | Citation of document, with indication, where appropriate, of the relevant passages | Relevant to claim No. |
| --- | --- | --- |
| X | HAMED Elham O. et al. "Significance of HE4 estimation in comparison with CA125 in diagnosis of ovarian cancer and assessment of treatment response". Diagnostic Pathology, 2013; 8: 11, doi: 10.1186/1746-1596-8-11, introduction, p. 3, left col., p. 4, right col., table 1 | 1,5,6,8,10,11 |
| A | | 2-4,7,9,12 |
| X | BAKRANI Mahnaz et al. "Comparison of the Serum Level of Cancer Antigen 125 and Human Epididymis Protein 4 in Ovarian Cancer Patients and Healthy Groups in Isfahan City". Adv Biomed Res, 2017, 6: 124, doi: 10.4103/2277-9175.216778, introduction | 1,5,6,10,11 |

☐ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21 December 2020 (21.12.2020) | 14 January 2021 (14.01.2021) |

| Name and mailing address of the ISA/ RU | Authorized officer |
| --- | --- |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **B.I. POLOZHINTSEV.** Theory of Probability and Mathematical Statistics. Introduction to Mathematical Statistics: A Study Guide. SPb.: Publishing house of Polytechnic University, 2010, 95
- **SEVERIN SE ; MOSKALEVA EY ; SHMYREV II et al.** Alpha-fetoprotein-mediated targeting of anticancer drugs to tumor cells in vitro. *Biochem Mol Biol Int,* 1995, vol. 37, 385-392
- **CANCER V.** Compositions of Alpha-fetoprotein and inducers of apoptosis for the treatment of cancer. *Google Patents,* 2008, http://www.google.com.na/patents/US20080318840
- **JANNETA TCHERKASSOVA ; SERGEI TSURKAN ; GALINA SMIRNOVA ; JULIA BORISOVA ; RICARDO MORO ; HELEN TRESHALINA.** Binding characterization of the targeting drug AIMPILA to AFP receptors in human tumor xenographts. *Tumor Biology,* 2017, 1-11
- **SERGEI TSURKAN ; JANNETA TCHERKASSOVA ; VERA GORBUNOVA ; HELEN TRESHALINA ; ELENA YU ; GRIGORIEVA.** New drug AIMPILA targeted to AFP receptor: Oral anticancer therapy and biodistribution in vivo. *Journal of Clinical Oncology.,* 2018, vol. 36 (15), e24232
- Handbook of Experimental Immunology. Blackwell Science Inc, 1987
- Current Protocols in Molecular Biology. 1987
- **SPIRO, A. ; LOWE, M. ; BROWN, D.** A Bead-Based Method for Multiplexed Identification and Quantitation of DNA Sequences Using Flow Cytometry. *Appl. Env. Micro.,* 2000, vol. 66, 4258-4265
- **CROWTHER, J. R.** The ELISA guidebook. Humana Press, 2000
- **HARLOW, E. ; LANE, D.** Using antibodies: a laboratory manual. Cold Spring Harbor Laboratory Press, 1999